# EUROPEAN PATENT APPLICATION

(11) **EP 3 632 553 A1**
(43) Date of publication of application: **08.04.2020**
(21) Application number: 18809883.4
(22) Date of filing: 31.05.2018
(51) Int. Cl.: B01J 29/14, B01J 29/16, B01J 29/46, B01J 29/48, B01J 29/68, B01J 29/69, B01J 29/76, B01J 29/78, B01J 37/10, C07B 61/00, C07C 2/84, C07C 4/18, C07C 15/04, C07C 15/06, C07C 15/085, C07C 15/46

(54) **CATALYST STRUCTURE FOR AROMATIC HYDROCARBON PRODUCTION, AROMATIC HYDROCARBON PRODUCTION DEVICE PROVIDED WITH SAID CATALYST STRUCTURE FOR AROMATIC HYDROCARBON PRODUCTION, PRODUCTION METHOD OF CATALYST STRUCTURE FOR AROMATIC HYDROCARBON PRODUCTION, AND PRODUCTION METHOD OF AROMATIC HYDROCARBONS**

(30) Priority: 31.05.2017 JP 2017108614
(71) Applicant: Furukawa Electric Co., Ltd., Chiyoda-ku Tokyo 100-8322 (JP)
(72) Inventor: MASUDA, Takao, Hokkaido 0600808 (JP); NAKASAKA, Yuta, Hokkaido 0600808 (JP); YOSHIKAWA, Takuya, Hokkaido 06000808 (JP); KATO Sadahiro, Tokyo 100-8322 (JP); FUKUSHIMA Masayuki, Tokyo 100-8322 (JP); TAKAHASHI Hiroko, Tokyo 100-8322 (JP); BANBA Yuichiro, Tokyo 100-8322 (JP); SEKINE Kaori, Tokyo 100-8322 (JP)
(74) Representative: Gunzelmann, Rainer
(86) International application number: PCT/JP2018/021092
(87) International publication number: WO 2018/221704

(57) **Abstract**

Provided are a structured catalyst for aromatic hydrocarbon production and an aromatic hydrocarbon producing device including a structured catalyst for aromatic hydrocarbon production, in which a reduction in catalytic activity is suppressed and an aromatic hydrocarbon can be efficiently produced. A structured catalyst for aromatic hydrocarbon production, including:
a support of a porous framework composed of a zeolite-type compound; and
at least one catalytic substance present in the support,
in which the support has channels communicating with each other, and
the catalytic substance is made of metal nanoparticles and is present at least in the channels of the support.

## Description

### Technical Field

The present invention relates to a structured catalyst for aromatic hydrocarbon production, particularly a structured catalyst for aromatic hydrocarbon production for producing an aromatic hydrocarbon from a light hydrocarbon, an aromatic hydrocarbon producing device including the structured catalyst for aromatic hydrocarbon production, a method for producing a structured catalyst for aromatic hydrocarbon production, and a method for producing an aromatic hydrocarbon.

### Background Art

Aromatic hydrocarbons, especially benzene, toluene and xylene are useful compounds used as raw materials in various fields. Currently, aromatic hydrocarbons are usually produced from petroleum. However, since petroleum resources are limited, development of an alternative method for producing aromatic hydrocarbons is desired.

Aromatic hydrocarbons can be produced, for example, from light hydrocarbons by a dehydrogenation ring reaction. Non-Patent Document 1 discloses Mo/ZSM-5 as a catalyst used for producing benzene from methane.

### Citation List

### Non-Patent Document

Non-Patent Document 1: Eurasian Chem Tech Journal 12 (2010) p. 9-16

### Summary of Invention

### Technical Problem

However, in the Mo/ZSM-5 disclosed in Non-Patent Document 1, the particle size of the Mo particles supported on ZSM-5 is smaller than the pore diameter of ZSM-5, so there is concern that the Mo particles aggregate during the catalyst reaction and that catalytic activity will decline.

An object of the present invention is to provide a structured catalyst for aromatic hydrocarbon production that suppresses the decline in catalytic activity and allows efficient production of an aromatic hydrocarbon, an aromatic hydrocarbon producing device including the structured catalyst for aromatic hydrocarbon production, a method for producing a structured catalyst for aromatic hydrocarbon production, and a method for producing an aromatic hydrocarbon.

### Solution to Problem

As a result of diligent research to achieve the object described above, the present inventors have found that the structured catalyst for aromatic hydrocarbon production that can suppress the decline in catalytic activity and that allows efficient production of an aromatic hydrocarbon can be obtained by including:
a support of a porous framework composed of a zeolite-type compound; and
at least one catalytic substance present in the support,
in which the support has channels communicating with each other, and
the catalytic substance is made of metal nanoparticles and is present at least in the channels of the support;
and thus completed the present invention based on such finding.

In other words, the summary configurations of the present invention are as follows.
[1] A structured catalyst for aromatic hydrocarbon production, including:
   a support of a porous framework composed of a zeolite-type compound; and
   at least one catalytic substance present in the support,
   in which the support has channels communicating with each other, and
   the catalytic substance is made of metal nanoparticles and is present at least in the channels of the support.
[2] The structured catalyst for aromatic hydrocarbon production according to [1], in which the metal nanoparticles are nanoparticles composed of at least one metal selected from the group consisting of cobalt (Co), nickel (Ni), iron (Fe), copper (Cu), gold (Au), silver (Ag), platinum (Pt), palladium (Pd), rhodium (Rd), iridium (Ir), ruthenium (Ru), osmium (Os), and molybdenum (Mo).
[3] The structured catalyst for aromatic hydrocarbon production according to [1] or [2], in which the channels each have an enlarged pore portion, and the catalytic substance is at least embedded in the enlarged pore portion.
[4] The structured catalyst for aromatic hydrocarbon production according to [2], in which the enlarged pore portion communicates with a plurality of pores constituting any one of a one-dimensional pore, a two-dimensional pore, and a three-dimensional pore.
[5] The structured catalyst for aromatic hydrocarbon production according to [3] or [4], in which the average particle size of the metal nanoparticles is greater than an average inner diameter of the channels and is less than or equal to an inner diameter of the enlarged pore portion.
[6] The structured catalyst for aromatic hydrocarbon production according to any one of [1] to [5], in which a metal element (M) of the metal nanoparticles is contained in an amount of from 0.5 to 2.5 mass% relative to the structured catalyst for aromatic hydrocarbon production.
[7] The structured catalyst for aromatic hydrocarbon production according to any one of [1] to [6], in which the average particle size of the metal nanoparticles is from 0.08 nm to 30 nm.
[8] The structured catalyst for aromatic hydrocarbon production according to [7], in which the average particle size of the metal nanoparticles is from 0.35 nm to 11.0 nm.
[9] The structured catalyst for aromatic hydrocarbon production according to any one of [1] to [8], in which the ratio of the average particle size of the metal nanoparticles to the average inner diameter of the channels is from 0.05 to 300.
[10] The structured catalyst for aromatic hydrocarbon production according to [9], in which the ratio of the average particle size of the metal nanoparticles to the average inner diameter of the channels is from 0.1 to 30.
[11] The structured catalyst for aromatic hydrocarbon production according to [9] or [10], in which the ratio of the average particle size of the metal nanoparticles to the average inner diameter of the channels is from 1.4 to 3.6.
[12] The structured catalyst for aromatic hydrocarbon production according to any one of [1] to [11],
   in which the channels have any one of the one-dimensional pore, the two-dimensional pore, and the three-dimensional pore defined by a framework of the zeolite-type compound and the enlarged pore portion which is different from any one of the one-dimensional pore, the two-dimensional pore, and the three-dimensional pore,
   the average inner diameter of the channels is from 0.1 nm to 1.5 nm, and
   the inner diameter of the enlarged pore portion is from 0.5 nm to 50 nm.
[13] The structured catalyst for aromatic hydrocarbon production according to any one of [1] to [12], further including at least one other catalytic substance held on an outer surface of the support.
[14] The structured catalyst for aromatic hydrocarbon production according to [13], in which the content of the at least one catalytic substance present in the support is greater than that of the at least one other catalytic substance held on the outer surface of the support.
[15] The structured catalyst for aromatic hydrocarbon production according to any one of [1] to [14], in which the zeolite-type compound is a silicate compound.
[16] An aromatic hydrocarbon producing device including a structured catalyst for aromatic hydrocarbon production according to any one of [1] to [15].
[17] A method for producing a structured catalyst for aromatic hydrocarbon production, including:
   a step of calcination of calcinating a precursor material (B) obtained by impregnating a precursor material (A), for obtaining a support of a porous framework composed of zeolite-type compound, with a metal-containing solution;
   a step of hydrothermal treatment of hydrothermal-treating a precursor material (C) obtained by calcinating the precursor material (B); and
   a step of reduction treatment of the precursor material (C) that is hydrothermal-treated.
[18] The method for producing a structured catalyst for aromatic hydrocarbon production according to [17], in which from 50 to 500 mass% of a non-ionic surfactant is added to the precursor material (A) before the step of calcination.
[19] The method for producing a structured catalyst for aromatic hydrocarbon production according to [17] or [18], in which the precursor material (A) is impregnated with the metal-containing solution by adding the metal-containing solution to the precursor material (A) in multiple portions before the step of calcination.
[20] The method for producing a structured catalyst for aromatic hydrocarbon production according to any one of [17] to [19], in which in impregnating the precursor material (A) with the metal-containing solution before the step of calcination, the added amount of the metal-containing solution added to the precursor material (A), converted into a ratio of silicon (Si) constituting the precursor material (A) to a metal element (M) included in the metal-containing solution added to the precursor material (A) (a ratio of number of atoms Si/M), is adjusted to from 10 to 1000.
[21] The method for producing a structured catalyst for aromatic hydrocarbon production according to [17], in which in the step of hydrothermal treatment, the precursor material (C) and a structure directing agent are mixed.
[22] The method for producing a structured catalyst for aromatic hydrocarbon production according to [17], in which the step of hydrothermal treatment is performed in basic condition.
[23] The method of producing a structured catalyst for aromatic hydrocarbon production according to [17], further including a step of carbonizing the structured catalyst for aromatic hydrocarbon production.
[24] A method for producing an aromatic hydrocarbon, including: adding a methane-containing gas to a structured catalyst for aromatic hydrocarbon production according to any one of [1] to [12].
[25] The method for producing an aromatic hydrocarbon according to [24], including the step of carbonizing the metal nanoparticles; and a step of adding a methane-containing gas to the structured catalyst.
[26] A method for producing an aromatic hydrocarbon, including treating a methane-containing gas with the aromatic hydrocarbon producing device according to [16].

### Advantageous Effects of Invention

According to the present invention, provided are a structured catalyst for aromatic hydrocarbon production that suppresses the decline in catalytic activity and allows efficient production of an aromatic hydrocarbon from, for example, a light hydrocarbon; and an aromatic hydrocarbon producing device including the structured catalyst for aromatic hydrocarbon production.

### Brief Description of Drawings

FIG. 1 is a diagram schematically illustrating a structured catalyst for aromatic hydrocarbon production according to an embodiment of the present invention so that the inner structure can be understood. FIG. 1A is a perspective view (partially shown in cross-section), and FIG. 1B is a partially enlarged cross-sectional view.
FIG. 2 is a partial enlarged cross-sectional view for explaining an example of the function of the structured catalyst for aromatic hydrocarbon production of FIG. 1. FIG. 2A is a diagram illustrating sieving capability, and FIG. 2B is a diagram explaining catalytic capacity.
FIG. 3 is a flowchart illustrating an example of a method for producing the structured catalyst for aromatic hydrocarbon production of FIG. 1.
FIG. 4 is a schematic view illustrating a modified example of the structured catalyst for aromatic hydrocarbon production of FIG. 1.

### Description of Embodiments

Hereinafter, embodiments of the present invention will be described in detail with reference to drawings.

### Configuration of Structured Catalyst

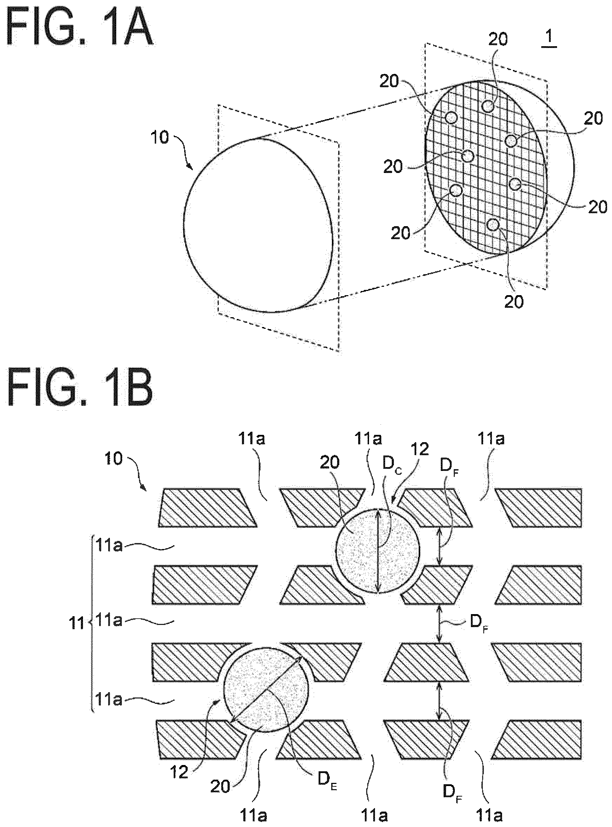

FIG. 1 is a diagram schematically illustrating a configuration of a structured catalyst for aromatic hydrocarbon production (hereinafter referred to simply as "structured catalyst") according to an embodiment of the present invention. In FIG. 1, A is a perspective view (partially shown in cross-section), and B is a partially enlarged cross-sectional view. Note that the structured catalyst in FIG. 1 is an example of the structured catalyst, and the shape, dimension, and the like of each of the configurations according to the present invention are not limited to those illustrated in FIG. 1.

As illustrated in FIG. 1A, a structured catalyst 1 includes a support 10 of a porous structure composed of a zeolite-type compound and at least one catalytic substance 20 present in the support 10.

In the structured catalyst 1, a plurality of the catalytic substances 20, 20, ... are embedded in the porous structure of the support 10. The catalytic substance 20 may be any substance as long as it has catalytic capacity (catalytic activity) and specifically is made of metal nanoparticles. The metal nanoparticles are described in detail below.

The support 10 has a porous structure, and as illustrated in FIG. 1B, a plurality of pores 11a, 11a, ... are preferably formed so as to have channels 11 communicating with each other. Here, the catalytic substance 20 is present at least in the channels 11 of the support 10 and is preferably held at least in the channels 11 of the support 10.

With such a configuration, movement of the catalytic substance 20 within the support 10 is restricted, and aggregation between the catalytic substances 20 and 20 is effectively prevented. As a result, the decrease in effective surface area of the catalytic substance 20 can be effectively suppressed, and the catalytic activity of the catalytic substance 20 lasts for a long period of time. In other words, according to the structured catalyst 1, the decline in catalytic activity due to aggregation of the catalytic substance 20 can be suppressed, and the life time of the structured catalyst 1 can be extended. In addition, due to the long life time of the structured catalyst 1, the replacement frequency of the structured catalyst 1 can be reduced, and the amount of waste of the used structured catalyst 1 can be significantly reduced and thereby can save resources.

Typically, when the structured catalyst is used in a fluid, it can be subjected to external forces from the fluid. In this case, if the catalytic substance is only held in the state of attachment to the outer surface of the support 10, there is a problem of easy detachment from the outer surface of the support 10 due to the influence of external force from the fluid. In contrast, in the structured catalyst 1, the catalytic substance 20 is held at least in the channels 11 of the support 10, and therefore, even if subjected to an external force caused by a fluid, the catalytic substance 20 is less likely to detach from the support 10. That is, when the structured catalyst 1 is in the fluid, the fluid flows into the channels 11 from the pores 11a of the support 10, so the speed of the fluid flowing through the channels 11 is slower than the speed of the fluid flowing on the outer surface of the support 10 due to the flow path resistance (frictional force). Due to the influence of such flow path resistance, the pressure experienced by the catalytic substance 20 held in the channels 11 from the fluid is lower than the pressure at which the catalytic substance is received from the fluid outside of the support 10. As a result, detachment of the catalytic substance 20 present in the support 11 can be effectively suppressed, and the catalytic activity of the catalytic substance 20 can be stably maintained over a long period of time. Note that the flow path resistance as described above is thought to be larger as the channels 11 of the support 10 have a plurality of bends and branches and as the interior of the support 10 becomes a more complex three-dimensional structure.

Preferably, the channels 11 have any one of a one-dimensional pore, a two-dimensional pore, and a three-dimensional pore defined by a framework of the zeolite-type compound; and an enlarged pore portion 12 which is different from any one of the one-dimensional pore, the two-dimensional pore, and the three-dimensional pore. In this case, the catalytic substance 20 is preferably present at least in the enlarged pore portion 12 and is more preferably embedded at least in the enlarged pore portion 12. Here, the "one-dimensional pore" refers to a tunnel-type or cage-type pore forming a one-dimensional channel; or a plurality of tunnel-type or cage-type pores (a plurality of one-dimensional channels) forming a plurality of one-dimensional channels. Also, the "two-dimensional pore" refers to a two-dimensional channel in which a plurality of one-dimensional channels is connected two-dimensionally. The "three-dimensional pore" refers to a three-dimensional channel in which a plurality of one-dimensional channels are connected three-dimensionally.

As a result, the movement of the catalytic substance 20 within the support 10 is further restricted, and it is possible to further effectively prevent detachment of the catalytic substance 20 and aggregation between the catalytic substances 20, 20. Embedding refers to a state in which the catalytic substance 20 is included in the support 10. At this time, the catalytic substance 20 and the support 10 need not necessarily be in direct contact with each other, but the catalytic substance 20 may be indirectly held by the support 10 with other substances (e.g., a surfactant, etc.) interposed between the catalytic substance 20 and the support 10.

Although FIG. 1B illustrates the case in which the catalytic substance 20 is embedded in the enlarged pore portion 12, the catalytic substance 20 is not limited to this configuration only, and the catalytic substance 20 may be held in the channels 11 with a portion thereof protruding outward of the enlarged pore portion 12. Furthermore, the catalytic substance 20 may be partially embedded in a portion of the channels 11 other than the enlarged pore portion 12 (for example, an inner wall portion of the channels 11) or may be held by fixing or the like, for example.

Additionally, the enlarged pore portion 12 preferably communicates with the plurality of pores 11a, 11a constituting any one of the one-dimensional pore, the two-dimensional pore, and the three-dimensional pore. As a result, a separate channel different from the one-dimensional pore, the two-dimensional pore, or the three-dimensional pore is provided in the support 10, so the function of the catalytic substance 20 can be further exhibited.

Additionally, the channels 11 are formed three-dimensionally by including a branch portion or a merging portion within the support 10, and the enlarged pore portion 12 is preferably provided in the branch portion or the merging portion of the channels 11.

An average inner diameter D_{F} of the channels 11 formed in the support 10 is calculated from the average value of the short diameter and the long diameter of the pore 11a constituting any one of the one-dimensional pore, the two-dimensional pore, and the three-dimensional pore. For example, it is from 0.1 nm to 1.5 nm and preferably from 0.5 nm to 0.8 nm. An inner diameter D_{E} of the enlarged pore portion 12 is from 0.5 nm to 50 nm, for example. The inner diameter D_{E} is preferably from 1.1 nm to 40 nm and more preferably from 1.1 nm to 3.3 nm. For example, the inner diameter D_{E} of the enlarged pore portion 12 depends on the pore diameter of a precursor material (A) described below and an average particle size D_{C} of the catalytic substance 20 to be embedded. The inner diameter D_{E} of the enlarged pore portion 12 is sized so that the enlarged pore portion 12 is able to embed the catalytic substance 20.

The support 10 is composed of a zeolite-type compound. Examples of zeolite-type compounds include zeolite analog compounds such as zeolites (alminosilicate salts), cation exchanged zeolites, silicate compounds such as silicalite, alminoborate salts, alminoarsenate salts, and germanate salts; and phosphate-based zeolite analog materials such as molybdenum phosphate. Among these, the zeolite-type compound is preferably a silicate compound.

The framework of the zeolite-type compound is selected from FAU type (Y type or X type), MTW type, MFI type (ZSM-5), FER type (ferrierite), LTA type (A type), MWW type (MCM-22), MOR type (mordenite), LTL type (L type), and BEA type (beta type). Preferably, it is MFI type and more preferably ZSM-5. A plurality of pores having a pore diameter corresponding to each framework is formed in the zeolite-type compound. For example, the maximum pore diameter of MFI type is 0.636 nm (6.36 Å), and the average pore diameter is 0.560 nm (5.60 Å).

In the following, the catalytic substance 20 will be described in detail.

The catalytic substance 20 is made of metal nanoparticles. The metal nanoparticles may be held in the channels 11 in the state of primary particles or held in the channels 11 in the state of secondary particles formed by aggregation of primary particles. In both cases, the average particle size D_{C} of the metal nanoparticles is preferably greater than the average inner diameter D_{F} of the channels 11 and is less than or equal to the inner diameter D_{E} of the enlarged pore portion 12 (D_{F} < D_{C} ≤ D_{E}). Such catalytic substance 20 is suitably embedded in the enlarged pore portion 12 within the channels 11, and the movement of the catalytic substance 20 within the support 10 is restricted. Thus, even if the catalytic substance 20 is subjected to an external force from a fluid, movement of the catalytic substance 20 within the support 10 is suppressed, and it is possible to effectively prevent contact between the catalytic substances 20, 20, ... embedded in the enlarged pore portions 12, 12, ... dispersed in the channels 11 of the support 10.

In addition, the average particle size D_{C} of the metal nanoparticles is preferably from 0.08 nm to 30 nm, more preferably 0.08 nm or higher and less than 25 nm, further preferably from 0.35 nm to 11.0 nm, and particularly preferably from 0.8 nm to 2.7 nm for primary particles and secondary particles. Furthermore, the ratio (D_{C}/D_{F}) of the average particle size D_{C} of the metal nanoparticles to the average inner diameter D_{F} of the channels 11 is preferably from 0.05 to 300, more preferably from 0.1 to 30, even more preferably from 1.1 to 30, and particularly preferably from 1.4 to 3.6.

When the catalytic substance 20 is made of metal nanoparticles, a metal element (M) of the metal nanoparticles is preferably contained in from 0.5 to 2.5 mass% relative to the structured catalyst 1 and more preferably from 0.5 to 1.5 mass% relative to the structured catalyst 1. For example, when the metal element (M) is Co, the content of the Co element (mass%) is expressed as [(mass of Co element)/ (mass of all elements in the structured catalyst 1)] × 100.

The metal nanoparticles only need to be constituted by a metal that is not oxidized and may be constituted by a single metal or a mixture of two or more types of metals, for example. Note that in the present specification, the "metal" constituting the metal nanoparticles (as the raw material) refers to an elemental metal containing one type of metal element (M) and a metal alloy containing two or more types of metal elements (M), and the term is a generic term for a metal containing one or more metal elements.

Examples of the metal include platinum (Pt), palladium (Pd), ruthenium (Ru), nickel (Ni), cobalt (Co), molybdenum (Mo), tungsten (W), iron (Fe), chromium (Cr), cerium (Ce), copper (Cu), magnesium (Mg), aluminum (Al), gold (Au), silver (Ag), iridium (Ir), ruthenium (Ru), rhodium (Rd), and osmium (Os). Preferably, any one of the metals described above is the major component. In particular, from the viewpoint of a catalytic activity, the metal nanoparticles are preferably nanoparticles composed of at least one metal selected from the group consisting of cobalt (Co), nickel (Ni), iron (Fe), copper (Cu), gold (Au), silver (Ag), platinum (Pt), palladium (Pd), rhodium (Rd) iridium (Ir), ruthenium (Ru), osmium (Os), and molybdenum (Mo). Among these, the metal nanoparticles are particularly preferably molybdenum and molybdenum alloys and most preferably molybdenum and molybdenum alloys that have been carbonized.

Furthermore, the ratio of silicon (Si) constituting the support 10 to the metal element (M) constituting the metal nanoparticles (the ratio of number of atoms Si/M) is preferably from 10 to 1000 and more preferably from 50 to 200. In a case where the ratio is greater than 1000, the activity is low, and the action as the catalytic substance may not be sufficiently obtained. On the other hand, in a case where the ratio is smaller than 10, the proportion of the metal nanoparticles becomes too large, and the strength of the support 10 tends to decrease. Note that the metal nanoparticles 20 herein refer to nanoparticles present or supported in the support 10 and do not include metal nanoparticles adhered to the outer surface of the support 10.

### Function of Structured Catalyst

As described above, the structured catalyst 1 includes the support 10 of a porous structure and at least one catalytic substance 20 present in the support 10. The structured catalyst 1 exhibits a catalytic capacity according to the function of the catalytic substance 20 upon contact of the catalytic substance 20 present in the support 10 with a fluid. Specifically, the fluid in contact with an outer surface 10a of the structured catalyst 1 flows into the support 10 through the pore 11a formed in the outer surface 10a and guided into the channels 11, moves through the channels 11, and exits to the exterior of the structured catalyst 1 through the other pore 11a. In the pathway through which the fluid travels through the channels 11, the contact with the catalytic substance 20 held in the channels 11 results in a catalytic reaction according to the catalytic substance 20. In addition, the structured catalyst 1 has molecular sieving capability due to the porous structure of the support.

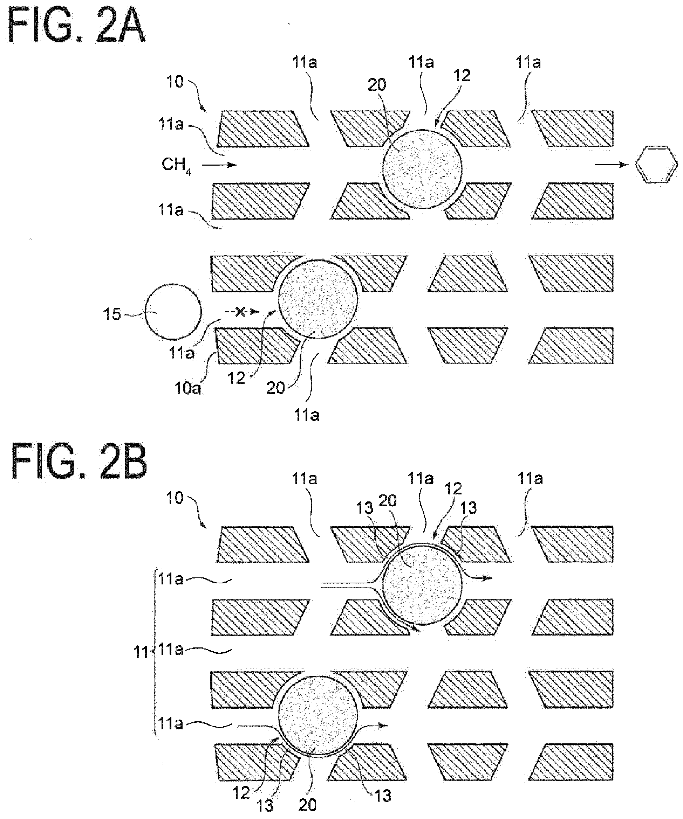

First, the molecular sieving capability of the structured catalyst 1 is described using FIG. 2A in which the fluid is, for example, a methane-containing gas. Note that "methane-containing gas" refers to a mixed gas containing methane and a gas other than methane.

As illustrated in FIG. 2A, methane (CH₄) composed of molecules having a size that is less than or equal to the pore diameter of the pore 11a, in other words, less than or equal to the inner diameter of the channels 11, can flow into the support 10. On the other hand, a gas component 15 composed of molecules having a size exceeding the pore diameter of the pore 11a cannot flow into the support 10. In this way, when the fluid contains a plurality of types of compounds, the reaction of the gas component 15 that cannot flow into the support 10 can be restricted, while methane capable of flowing into the support 10 is allowed to react.

Of the compounds produced in the support 10 by the reaction, only compounds composed of molecules having a size less than or equal to the pore diameter of the pore 11a can exit through the pore 11a to the exterior of the support 10 and are obtained as reaction products. On the other hand, a compound that cannot exit to the exterior of the support 10 from the pore 11a can be released to the exterior of the support 10 after being converted into a compound composed of molecules sized to be able to exit to the exterior of the support 10. In this way, a specified reaction product can be selectively obtained by using the structured catalyst 1. In the present embodiment, benzene is obtained as a reaction product by a dehydrocyclization reaction of methane.

In the structured catalyst 1, as illustrated in FIG. 2B, the catalytic substance 20 is embedded in the enlarged pore portion 12 of the channels 11. When the average particle size D_{C} of the catalytic substance 20 (metal nanoparticles) is larger than the average inner diameter D_{F} of the channels 11 and smaller than the inner diameter D_{E} of the enlarged pore portion 12 (D_{F} < D_{C} < D_{E}), a small channel 13 is formed between the catalytic substance 20 and the enlarged pore portion 12. Thus, as indicated by the arrow in FIG. 2B, the fluid that has flown into the small channel 13 comes into contact with the catalytic substance 20. Because each catalytic substance 20 is embedded in the enlarged pore portion 12, movement within the support 10 is restricted. As a result, aggregation between the catalytic substances 20 in the support 10 is prevented. As a result, a large contact area between the catalytic substance 20 and the fluid can be stably maintained.

The production of benzene from methane as a raw material is an example, and the use of the structured catalyst 1 allows the production of various aromatic hydrocarbons from light hydrocarbons. The light hydrocarbon is, for example, a hydrocarbon having a carbon number of 1 to 6 and is preferably an alkane having a carbon number of 1 to 6. The light hydrocarbon as a raw material may be constituted by a single compound or a mixture.

The method for producing an aromatic hydrocarbon from a methane-containing gas may include the steps of, for example, carbonizing the structured catalyst 1 with a methane gas or the like, followed by feeding a methane-containing gas to the carbonized structured catalyst 1.

### Method for Producing Structured Catalyst

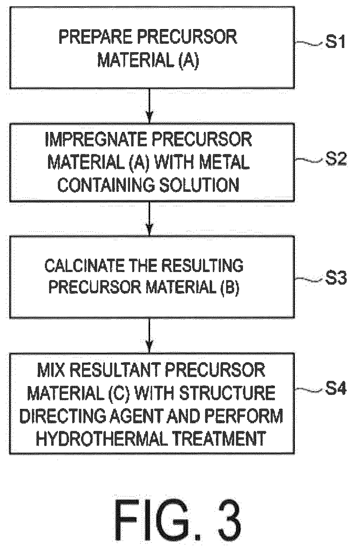

FIG. 3 is a flowchart illustrating a method for producing the structured catalyst 1 of FIG. 1 An example of the method for producing the structured catalyst will be described below, in which the catalytic substance 20 present in the support is made of metal nanoparticles.

### Step S1: Step of Preparation

As illustrated in FIG. 3, firstly, the precursor material (A) for obtaining a support of a porous structure composed of a zeolite-type compound is prepared. The precursor material (A) is preferably a regular mesopore material and can be appropriately selected according to the type (composition) of the zeolite-type compound constituting the support of the structured catalyst.

Here, when the zeolite-type compound constituting the support of the structured catalyst is a silicate compound, the regular mesopore material is preferably a compound including a Si-O skeleton in which pores having a pore diameter of from 1 to 50 nm are uniformly sized and regularly developed one-dimensionally, two-dimensionally, or three-dimensionally. While such a regular mesopore material is obtained as a variety of synthetic materials depending on the synthetic conditions. Specific examples of the synthetic material include SBA-1, SBA-15, SBA-16, KIT-6, FSM-16, and MCM-41. Among them, MCM-41 is preferred. Note that the pore diameter of SBA-1 is from 10 to 30 nm, the pore diameter of SBA-15 is from 6 to 10 nm, the pore diameter of SBA-16 is 6 nm, the pore diameter of KIT-6 is 9 nm, the pore diameter of FSM-16 is from 3 to 5 nm, and the pore diameter of MCM-41 is from 1 to 10 nm. Examples of such a regular mesopore material include mesoporous silica, mesoporous aluminosilicate, and mesoporous metallosilicate.

The precursor material (A) may be a commercially available product or a synthetic product. When the precursor material (A) is synthesized, it can be synthesized by a known method for synthesizing a regular mesopore material. For example, a mixed solution including a raw material containing the constituent elements of the precursor material (A) and a molding agent for defining the structure of the precursor material (A) is prepared, and the pH is adjusted as necessary to perform hydrothermal treatment (hydrothermal synthesis). Thereafter, the precipitate (product) obtained by hydrothermal treatment is recovered (e.g., filtered), washed and dried as necessary, and then calcinated to obtain the precursor material (A) which is a powdered regular mesopore material. Here, examples of the solvent of the mixed solution include water, organic solvents such as alcohols, and mixed solvents thereof. In addition, the raw material is selected according to the type of the support, and examples thereof include silica agents such as tetraethoxysilane (TEOS); fumed silica; and quartz sand. In addition, various types of surfactants, block copolymers, and the like can be used as the molding agent, and it is preferably selected depending on the type of the synthetic materials of the regular mesopore material. For example, a surfactant such as hexadecyltrimethylammonium bromide is preferable when producing MCM-41. The hydrothermal treatment can be performed at from 0 to 2000 kPa at from 80 to 800°C for from 5 hours to 240 hours in a sealed container. For example, the calcination treatment can be performed in air, at from 350 to 850°C for from 2 hours to 30 hours.

### Step S2: Step of Impregnation

The prepared precursor material (A) is then impregnated with the metal-containing solution to obtain a precursor material (B).

The metal-containing solution is a solution containing a metal component (for example, a metal ion) corresponding to the metal element (M) constituting the metal nanoparticles and can be prepared, for example, by dissolving a metal salt containing the metal element (M) in a solvent. Examples of the metal salt include chlorides, hydroxides, oxides, sulfates, nitrates, and alkali metal salts. Among these, nitrates are preferable. Examples of the solvent include water, organic solvents such as alcohols, and mixed solvents thereof.

The method for impregnating the precursor material (A) with the metal-containing solution is not particularly limited; however, for example, the metal-containing solution is preferably added in portions in a plurality of times while mixing the powdered precursor material (A) before the step of calcination described below. In addition, the surfactant is preferably added to the precursor material (A) as the additive before adding the metal-containing solution to the precursor material (A) from the perspective of allowing the metal-containing solution to enter the pores of the precursor material (A) more easily. It is believed that such additives serve to cover the outer surface of the precursor material (A) and inhibit the subsequently added metal-containing solution from adhering to the outer surface of the precursor material (A), making it easier for the metal-containing solution to enter the pores of the precursor material (A).

Examples of the additive include non-ionic surfactants such as polyoxyethylene alkyl ethers such as polyoxyethylene oleyl ether; and polyoxyethylene alkylphenyl ether. It is believed that these surfactants do not adhere to the interior of the pores because their molecular size is large, cannot enter the pores of the precursor material (A), and will not interfere with the penetration of the metal-containing solution into the pores. As the method for adding the non-ionic surfactant, for example, it is preferable to add from 50 to 500 mass% of the non-ionic surfactant to the precursor material (A) before the step of calcination described below. In a case where the added amount of the non-ionic surfactant to the precursor material (A) is less than 50 mass%, the aforementioned suppressing action will not easily occur, and when more than 500 mass% of the non-ionic surfactant is added to the precursor material (A), the viscosity is too high, which is not preferable. Thus, the added amount of the non-ionic surfactant to the precursor material (A) is a value within the range described above.

Furthermore, the added amount of the metal-containing solution added to the precursor material (A) is preferably adjusted as appropriate in consideration of the amount of the metal element (M) contained in the metal-containing solution with which the precursor material (A) is impregnated (that is, the amount of the metal element (M) present in the precursor material (B)). For example, before the step of calcination described below, the added amount of the metal-containing solution added to the precursor material (A), converted into a ratio of silicon (Si) constituting the precursor material (A) to the metal element (M) included in the metal-containing solution added to the precursor material (A) (the ratio of number of atoms Si/M), is preferably adjusted to from 10 to 1000 and more preferably from 50 to 200. For example, before adding the metal-containing solution to the precursor material (A), when a surfactant is added to the precursor material (A) as an additive and when the added amount of the metal-containing solution added to the precursor material (A), converted into the ratio of number of atoms Si/M, is from 50 to 200, from 0.5 to 2.5 mass% of the metal element (M) of the metal nanoparticles can be included in the structured catalyst. In the state of the precursor material (B), the amount of the metal element (M) present within the pores is generally proportional to the added amount of the metal-containing solution added to the precursor material (A) in a case where the metal concentration of the metal-containing solution, the presence or absence of additives, and other conditions such as temperature, pressure, and the like are the same. The amount of metal element (M) present in the precursor material (B) is also in a proportional relationship to the amount of metal element constituting the metal nanoparticles present in the support of the structured catalyst. Thus, by controlling the added amount of the metal-containing solution added to the precursor material (A) to the range described above, the pores of the precursor material (A) can be sufficiently impregnated with the metal-containing solution, and thus the amount of the metal nanoparticles present in the support of the structured catalyst can be adjusted.

After impregnating the precursor material (A) with the metal-containing solution, a washing treatment may be performed as necessary. Examples of the washing solution include water, organic solvents such as alcohols, and mixed solvents thereof. Furthermore, the precursor material (A) is preferably impregnated with the metal-containing solution, and after the washing treatment is performed as necessary, the precursor material (A) is further subjected to drying treatment. Drying treatments include overnight natural drying and high temperature drying at 150°C or lower. Note that when calcination treatment described below is performed in the state in which there is a large amount of moisture remaining in the metal-containing solution and the washing solution in the precursor material (A), the skeletal structure of the regular mesopore material of the precursor material (A) may be broken, and thus it is preferable to dry them sufficiently.

### Step S3: Step of Calcination

Next, a precursor material (C) is obtained by calcinating the precursor material (B) obtained by impregnating the precursor material (A), for obtaining the support of a porous framework composed of a zeolite-type compound, with the metal-containing solution.

For example, the calcination treatment is preferably performed in air, at from 350 to 850°C for from 2 hours to 30 hours. The metal component that has entered into the pores of the regular mesopore material undergoes crystal growth by such calcination treatment, and metal nanoparticles are formed in the pores.

### Step S4: Step of Hydrothermal Treatment

A mixed solution of the precursor material (C) and a structure directing agent is then prepared, and the precursor material (C) obtained by calcinating the precursor material (B) is hydrothermal-treated to obtain a structured catalyst.

The structure directing agent is a molding agent for directing the skeletal structure of the support of the structured catalyst and may be, for example, a surfactant. The structure directing agent is preferably selected according to the skeletal structure of the support of the structured catalyst and is preferably a surfactant such as tetramethyl ammonium bromide (TMABr), tetraethylammonium bromide (TEABr), or tetrapropylammonium bromide (TPABr).

The mixing of the precursor material (C) and the structure directing agent may be performed during the step of hydrothermal treatment or may be performed before the step of hydrothermal treatment. Furthermore, the method for preparing the mixed solution is not particularly limited, and the precursor material (C), the structure directing agent, and the solvent may be mixed simultaneously, or each of the dispersion solutions may be mixed after the precursor material (C) and the structure directing agent are each dispersed in individual solutions. Examples of the solvent include water, organic solvents such as alcohols, and mixed solvents thereof. In addition, it is preferable that the pH of the mixed solution is adjusted using an acid or a base before performing the hydrothermal treatment.

The hydrothermal treatment can be performed by a known method. For example, the hydrothermal treatment can be preferably performed at from 0 to 2000 kPa at from 80 to 800°C for from 5 hours to 240 hours in a sealed container. Furthermore, the hydrothermal treatment is preferably performed under basic condition. Although the reaction mechanism here is not necessarily clear, by performing hydrothermal treatment using the precursor material (C) as a raw material, the skeletal structure of the regular mesopore material of the precursor material (C) becomes increasingly disrupted. However, the action of the structure directing agent forms a new skeletal structure (porous structure) of the support of the structured catalyst while maintaining the position of the metal nanoparticles within the pores of the precursor material (C). The structured catalyst obtained in this way includes a support of a porous structure and metal nanoparticles present in the support, the support has channels in which a plurality of pores communicate with each other by the porous structure, and at least a portion of the metal nanoparticles is held in the channels of the support.

Furthermore, in the present embodiment, in the step of hydrothermal treatment, a mixed solution in which the precursor material (C) and the structure directing agent are mixed is prepared, and the precursor material (C) is subjected to hydrothermal treatment. Not only limited to this, the precursor material (C) may be subjected to hydrothermal treatment without mixing the precursor material (C) and the structure directing agent.

The precipitate (structured catalyst) obtained after hydrothermal treatment is preferably washed, dried, and calcinated as necessary after recovery (e.g., filtration). Examples of the washing solution that can be used include water, an organic solvent such as alcohol, or a mixed solution thereof. Drying treatments include overnight natural drying and high temperature drying at 150°C or lower. Note that when calcination treatment is performed in the state in which there is a large amount of moisture remaining in the precipitate, the skeletal structure as a support of the structured catalyst may be broken, and thus it is preferable to dry the precipitate sufficiently. For example, the calcination treatment can be also performed in air, at from 350 to 850°C for from 2 hours to 30 hours. Such calcination treatment burns out the structure directing agent that has been attached to the structured catalyst. Furthermore, the structured catalyst can be used as is without subjecting the recovered precipitate to calcination treatment, depending on the intended use. For example, in a case where the environment in which the structured catalyst is used is a high temperature environment of an oxidizing condition, exposing the structured catalyst to the usage environment for a period of time allows the structure directing agent to be burned out. In this case, the same structured catalyst as when subjected to calcination treatment is obtained, so it is not necessary to perform the calcination treatment.

The producing method described above is an example in which the metal element (M) contained in the metal-containing solution that impregnates the precursor material (A) is a metal species resistant to oxidation (e.g., a noble metal).

When the metal element (M) contained in the metal-containing solution that impregnates the precursor material (A) is an easily oxidized metal species (e.g., Fe, Co, Ni, or Cu), the hydrothermal treatment described above is preferably followed by reduction treatment. When the metal element (M) contained in the metal-containing solution is an easily oxidized metal species, the metal component is oxidized by the heat treatment in the steps (steps S3 to S4) after the step of impregnation (step S2). Therefore, metal oxide nanoparticles are present in the support formed in the step of hydrothermal treatment (step S4). Therefore, in order to obtain a structured catalyst in which metal nanoparticles are present in a support, the precipitate recovered after the hydrothermal treatment is preferably subjected to calcination treatment and further to reduction treatment in reducing gas condition such as hydrogen gas. In reduction treatment, the metal oxide nanoparticles present in the support are reduced, and metal nanoparticles corresponding to the metal element (M) constituting the metal oxide nanoparticles are formed. As a result, a structured catalyst in which metal nanoparticles are present in a support is obtained. Note that the reduction treatment may be performed as necessary. For example, when the environment in which the structured catalyst is used is reducing gas condition, the metal oxide nanoparticles are reduced by exposure to the usage environment for a certain period of time. In this case, the same structured catalyst as that obtained through reduction treatment is obtained, so it is not necessary to perform the reduction treatment.

### Modified Example of Structured Catalyst 1

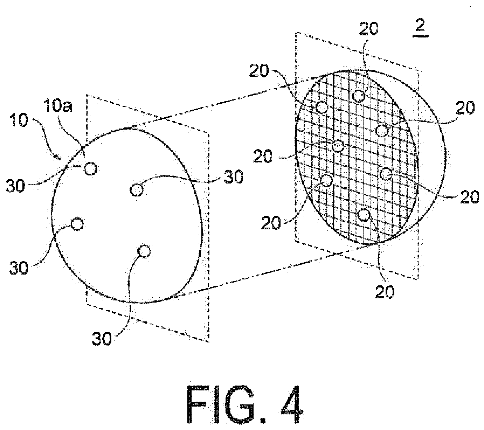

FIG. 4 is a schematic view illustrating a modified example of the structured catalyst 1 in FIG. 1.

Although the structured catalyst 1 of FIG. 1 illustrates the case in which it includes the support 10 and the catalytic substance 20 present in the support 10, the structured catalyst 1 is not limited to this configuration. For example, as illustrated in FIG. 4, the structured catalyst 2 may further include other catalytic substance 30 held on the outer surface 10a of the support 10.

This catalytic substance 30 is a substance that exhibits one or more catalytic capacities. The catalytic capacities of the other catalytic substance 30 may be the same or different from the catalytic capacity of the catalytic substance 20. Also, if both of the catalytic substances 20, 30 are substances having the same catalytic capacity, the material of the other catalytic substance 30 may be the same as or different from the material of the catalytic substance 20. According to this configuration, the content of catalytic substances held in a structured catalyst 2 can be increased, and the catalytic activity of the catalytic substances can be further accelerated.

In this case, the content of the catalytic substance 20 present in the support 10 is preferably greater than that of the other catalytic substance 30 held on the outer surface 10a of the support 10. As a result, the catalytic capacity of the catalytic substance 20 held in the support 10 becomes dominant, and catalytic capacity of the catalytic substance is stably exhibited.

Hereinbefore, the structured catalyst according to the present embodiments has been described, but the present invention is not limited to the above embodiments, and various modifications and changes are possible on the basis of the technical concept of the present invention.

For example, an aromatic hydrocarbon producing device including the structured catalyst may be provided. The producing device includes, for example, a liquid-phase fluidized bed reaction column. The use of the structured catalyst in a catalytic reaction using a producing device having such a configuration allows achievement of the same effects as described above, such as the production of an aromatic hydrocarbon compound from a methane-containing gas.

### Examples

### Example 1 to 384

### Synthesis of Precursor Material (A)

A mixed aqueous solution was prepared by mixing a silica agent (tetraethoxysilane (TEOS), available from Wako Pure Chemical Industries, Ltd.) and a surfactant as the molding agent. The pH was adjusted as appropriate, and hydrothermal treatment was performed at from 80 to 350°C for 100 hours in a sealed container. Thereafter, the produced precipitate was filtered out, washed with water and ethanol, and then calcinated in air at 600°C for 24 hours to obtain the precursor material (A) of the type and having the pore diameter shown in Tables 1 to 8. Note that the following surfactant was used in accordance with the type of the precursor material (A) ("precursor material (A): surfactant").
- MCM-41: Hexadecyltrimethylammonium bromide (CTAB) (available from Wako Pure Chemical Industries, Ltd.)
- SBA-1: Pluronic P123 (available from BASF)

### Making of Precursor Materials (B) and (C)

Next, a metal-containing aqueous solution was prepared by dissolving a metal salt containing the metal element (M) in water according to the metal element (M) constituting the metal nanoparticles of the type shown in Tables 1 to 8. Note that the metal salt was used in accordance with the type of metal nanoparticles ("metal nanoparticles: metal salt").
- Co: Cobalt nitrate (II) hexahydrate (available from Wako Pure Chemical Industries, Ltd.)
- Ni: Nickel nitrate (II) hexahydrate (available from Wako Pure Chemical Industries, Ltd.)
- Fe: Iron nitrate (III) nonahydrate (available from Wako Pure Chemical Industries, Ltd.)
- Mo: Molybdenum (VI) disodium dihydrate (available from Wako Pure Chemical Industries, Ltd.)

Next, a metal-containing aqueous solution was added to the powdered precursor material (A) in portions and dried at room temperature (20°C ± 10°C) for 12 hours or longer to obtain the precursor material (B).

Note that when the presence or absence of additives shown in Tables 1 to 8 is "yes", pretreatment, in which an aqueous solution of polyoxyethylene (15) oleyl ether (NIKKOL BO-15 V, available from Nikko Chemicals Co., Ltd.) is added as the additive to the precursor material (A) before adding the metal-containing aqueous solution, was performed, and then the metal-containing aqueous solution was added as described above. Note that when "no" is used in the presence or absence of additives, pretreatment with an additive such as that described above was not performed.

Furthermore, the added amount of the metal-containing aqueous solution added to the precursor material (A) was adjusted to become the value in Tables 1 to 8, converted into a ratio of silicon (Si) constituting the precursor material (A) to the metal element (M) included in the metal-containing aqueous solution (the ratio of number of atoms Si/M).

Next, the precursor material (B) impregnated with the metal-containing aqueous solution obtained as described above was calcinated in air at 600°C for 24 hours to obtain the precursor material (C).

The precursor material (C) obtained as described above and the structure directing agent shown in Tables 1 to 8 were mixed to produce a mixed aqueous solution and subjected to hydrothermal treatment in a sealed container under the conditions of at from 80 to 350°C and the pH and time shown in Tables 1 to 8. Thereafter, the produced precipitate was filtered off, washed with water, dried at 100°C for 12 hours or longer, and then calcinated in air at 600°C for 24 hours. In Examples 1 to 384, after the calcination treatment, the calcinated product was recovered and subjected to reduction treatment at 400°C for 350 minutes under the inflow of hydrogen gas, and a structured catalyst having the support shown in Tables 1 to 8 and metal nanoparticles (Co nanoparticles, Ni nanoparticles, Fe nanoparticles, or Mo nanoparticles) was obtained.

### Comparative Example 1

In Comparative Example 1, cobalt oxide powder (II, III) having an average particle size of 50 nm or less (available from Sigma-Aldrich Japan LLC.) was mixed with MFI type silicalite, and a structured catalyst in which cobalt oxide nanoparticles were attached as a catalytic substance to the outer surface of the silicalite as a skeletal body was obtained. MFI type silicalite was synthesized in the similar manner as in Examples 52 to 57 except for a step of adding a metal.

### Comparative Example 2

In Comparative Example 2, MFI type silicalite was synthesized in the similar manner as in Comparative Example 1 except that a step of attaching the cobalt oxide nanoparticles was omitted.

### Evaluation

Various characteristic evaluations were performed on the catalytic structural bodies of Examples 1 to 384 and the silicalite of Comparative Examples 1 and 2 under the conditions described below.

### [A] Cross-sectional Observation

Observation samples were made using a pulverization method for the catalytic structural bodies of Examples 1 to 384 and the silicalite of Comparative Example 1, and the cross-sectional observation was performed using a transmission electron microscope (TEM) (TITAN G2, available from FEI). As a result, it was confirmed that, in the structured catalyst of the example described above, the catalytic substance was present and held inside the support made of silicalite. On the other hand, in the structured catalyst of the comparative example, the catalytic substance was only attached to the outer surface of the support and was not present in the support.

In addition, of the examples described above, for the structured catalyst including Mo nanoparticles as the metal, the cross-section was cut by FIB (focused ion beam) processing, and cross-sectional element analysis was performed using SEM (SU8020, available from Hitachi High-Technologies Corporation) and EDX (X-Max, available from HORIBA, Ltd.). As a result, elements Mo were detected from inside the skeletal body.

It was confirmed that Mo nanoparticles were present in the support from the results of the cross-sectional observation using TEM and SEM/EDX.

### [B] Average Inner Diameter of the Channels of the Support and Average Particle Size of the Catalytic Substance

In the TEM image taken by the cross-sectional observation performed in evaluation [A] above, 500 channels of the support were randomly selected, the respective major diameter and the minor diameter were measured, the respective inner diameters were calculated from the average values (N = 500), and the average value of the inner diameter was determined to be the average inner diameter D_{F} of the channels of the support. In addition, for the catalytic substance, 500 catalytic substances were randomly selected from the TEM image, the respective particle sizes were measured (N = 500), and the average value thereof was determined to be the average particle size D_{C} of the catalytic substance. The results are shown in Tables 1 to 8.

Also, SAXS (small angle X-ray scattering) was used to analyze the average particle size and dispersion status of the catalytic substance. Measurements by SAXS were performed using a -SPring-8 beam line BL19B2. The obtained SAXS data was fitted with a spherical model using the Guinier approximation method, and the particle size was calculated. Particle size was measured for the structured catalyst in which the metal is Mo nanoparticles. Furthermore, as a comparative reference, a commercially available Fe₂O₃ nanoparticles (available from Wako Pure Chemical Industries, Ltd.) was observed and measured by SEM.

As a result, in commercial products, various sizes of Fe₂O₃ nanoparticles were randomly present in a range of particle sizes of approximately from 50 nm to 400 nm, whereas in the measurement results of SAXS, scattering peaks with particle sizes of 10 nm or less were also detected in the catalytic structural bodies of each example having an average particle size of from 1.2 nm to 2.0 nm determined from the TEM image. From the results of SAXS measurement and the SEM/EDX cross-sectional measurement, it was found that a catalytic substance having a particle size of 10 nm or less was present in the support in a markedly highly dispersed state with a uniform particle size.

### [C] Relationship between the Added Amount of the Metal-containing Solution and the Amount of Metal Embedded in the Skeletal Body

A structured catalyst in which metal nanoparticles were embedded in the support at an added amount of the ratio of number of atoms of Si/M = 50, 100, 200, 1000 (M = Co, Ni, Fe, and Mo) was produced, and then the amount of metal (mass%) that was embedded in the support of the structured catalyst produced at the above added amount was measured. Note that in the present measurement, the catalytic structural bodies having the ratio of number of atoms of Si/M = 100, 200, and 1000 were produced by adjusting the added amount of the metal-containing solution in the same manner as the catalytic structural bodies having the ratio of number of atoms of Si/M = 100, 200, and 1000 of Examples 1 to 384, and the catalytic structural bodies having the ratio of number of atoms of Si/M = 50 were produced in the same manner as the structured catalyst having the ratio of number of atoms of Si/M = 100, 200, and 1000, except that the added amount of the metal-containing solution was changed.

The amount of metal was quantified by ICP (radio frequency inductively coupled plasma) alone or in combination with ICP and XRF (fluorescence X-ray analysis). XRF (energy dispersive fluorescent x-ray analyzer "SEA1200VX", available from SII Nanotechnology) was performed under conditions of vacuum condition, an accelerating voltage 15 kV (using a Cr filter), or an accelerating voltage 50 kV (using a Pb filter).

XRF is a method for calculating the amount of metal present in terms of fluorescence intensity, and XRF alone cannot calculate a quantitative value (in terms of mass%). Therefore, the metal content of the structured catalyst to which the metal was added at Si/M = 100 was determined by ICP analysis, and the metal content of the structured catalyst in which the metal was added at Si/M = 50 and less than 100 was calculated based on XRF measurement results and ICP measurement results.

As a result, it was confirmed that the amount of the metal embedded in the structural body increased as the added amount of the metal-containing solution increased, at least within a range that the ratio of number of atom was within from 50 to 1000.

### [D] Performance Evaluation

The catalytic capacity of the catalytic substances was evaluated for the catalytic structural bodies of the examples and the silicalite of the comparative examples. The results are shown in Tables 1 to 8.

### (1) Catalytic Activity

The catalytic activity was evaluated under the following conditions:
First, 0.2 g of the structured catalyst was charged in a normal pressure flow reactor, and a decomposition reaction of butylbenzene (model material for heavy oil) was performed with nitrogen gas (N₂) as a carrier gas (5 ml/min) at 400°C for 2 hours.

After completion of the reaction, the compositions of the generated gas and the generated liquid that were collected were analyzed by gas chromatography mass spectrometry (GC/MS). Note that, as the analysis device for the generated gas, TRACE 1310GC (available from Thermo Fisher Scientific Inc., detector: thermal conductivity detector) was used, and as the analysis device for the generated liquid, TRACE DSQ (available from Thermo Fisher Scientific Inc., detector: mass detector, ionization method: EI (ion source temperature 250°C, MS transfer line temperature of 320°C, detector: thermal conductivity detector)) was used.

Based on the analysis results, the yield (mol%) of a compound having a molecular weight lower than that of butylbenzene (specifically, benzene, toluene, ethylbenzene, styrene, cumene, methane, ethane, ethylene, propane, propylene, butane, butene, and the like) was calculated. The yield of the compound was calculated as the percentage (mol%) of the total amount (mol) of the amount of the compound having a lower molecular weight than that of the butylbenzene contained in the generated liquid (mol%) relative to the amount of butylbenzene material (mol) before the reaction.

In the present example, when the yield of a compound having a molecular weight lower than that of butylbenzene contained in the product liquid was 20 mol% or more, it was evaluated that catalytic activity (resolution) is excellent, "A". When it was 15 mol% or more and less than 20 mol%, it was evaluated that catalytic activity is good, "B". When it was 10 mol% or more and less than 15 mol%, it was evaluated that catalytic activity is not good but is at a pass level (acceptable), "C". When it was less than 10 mol%, it was evaluated that catalytic activity is poor (unacceptable), "D".

The catalytic activity of the carbonized structured catalyst was evaluated under the following conditions.

First, of the catalytic structural bodies of Examples 1 to 384, the catalytic structural bodies containing molybdenum nanoparticles were charged into a normal pressure flow reactor, and carbonization treatment was performed for 30 minutes at 650°C while supplying methane. Thereafter, the temperature was raised to 800°C, and a methane dehydrogenase reaction was performed at SV 3000 mL/g/h. The normal pressure flow reactor used was Single micro-Reactor (Rx-3050SR, available from Frontier Laboratories Ltd.).

After completion of the reaction, the composition of the generated gas was analyzed by gas chromatography mass spectrometry (GC/MS). The analysis device for the generated gas was TRACE 1310GC (available from Thermo Fisher Scientific Inc., detector: thermal conductivity detector).

### Evaluation Criteria

When the methane conversion ratio was 10% or higher, the catalytic activity was evaluated to be excellent, "B", when 5% or more and less than 10%, the catalytic activity was evaluated to be not good but at a pass level (acceptable), "C", and when less than 5%, the catalytic activity was evaluated to be poor (unacceptable), "D". The results are shown in Table 9.

### (2) Durability (Life Time)

The durability was evaluated under the following conditions:
First, the structured catalyst used in evaluation (1) above was recovered and heated at 650°C for 12 hours to produce a structured catalyst after heating. Next, a decomposition reaction of butylbenzene (model material of heavy oil) was performed by the similar method as in evaluation (1) above using the obtained structured catalyst after heating, and component analysis of the generated gas and the generated liquid was performed in the similar manner as in the above evaluation (1).

Based on the analytical results, the yield (mol%) of the compound having a molecular weight lower than that of butylbenzene was determined in the similar manner as in evaluation (1) above. Furthermore, the degree of maintaining the yield of the above compound by the structured catalyst after heating was compared to the yield of the above compound by the structured catalyst before heating (the yield determined in evaluation (1) above). Specifically, the percentage (%) of the yield of the above compound obtained by the structured catalyst after heating (yield determined by evaluation (2) above) to the yield of the above compound by the structured catalyst before heating (yield determined by the present evaluation (1) above) was calculated.

In the present embodiment, when the yield of the compound (yield determined by the present evaluation (2) above) by the structured catalyst after heating was maintained at least 80% compared to the yield of the compound (yield determined by evaluation (1) above) by the structured catalyst before heating, it was evaluated that durability (heat resistance) is excellent, "A". When it was maintained 60% or more and less than 80%, it was evaluated that durability (heat resistance) is good, "B". When it was maintained 40% or more and less than 60%, it was evaluated that durability (heat resistance) is not good but is at a pass level (acceptable), "C". When it was reduced below 40%, it was evaluated that durability (heat resistance) is poor (unacceptable), "D".

Comparative Examples 1 and 2 were also subjected to the same performance evaluations as those in the evaluations (1) and (2) above. Comparative Example 2 is the support itself and includes no catalytic substance. Therefore, in the performance evaluation described above, only the support of Comparative Example 2 was charged in place of the structured catalyst. The results are shown in Table 8.

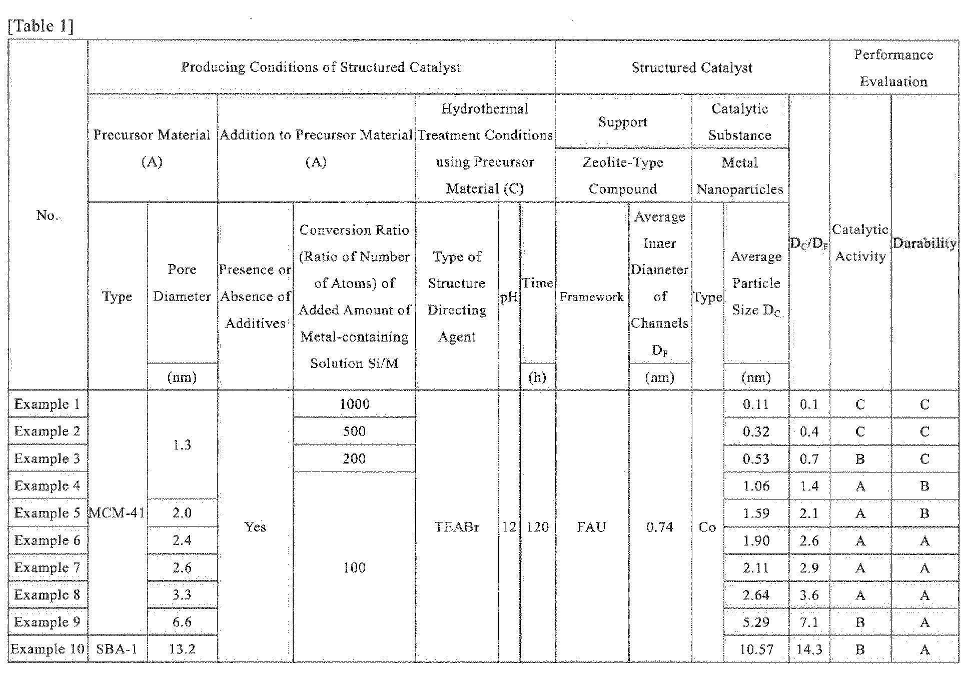

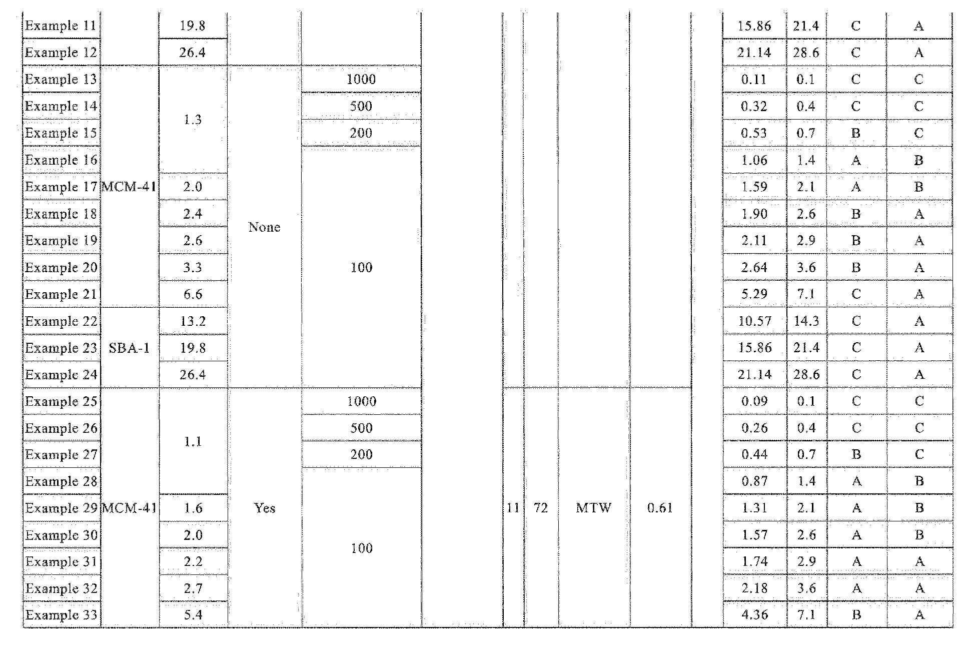

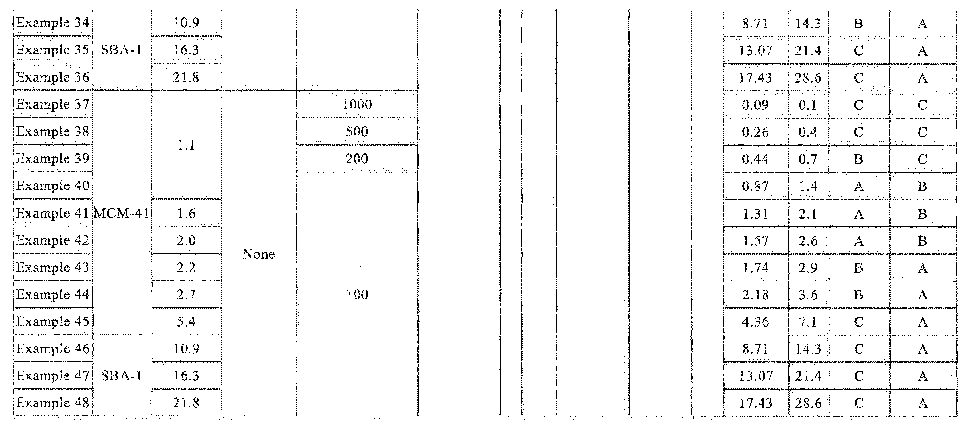

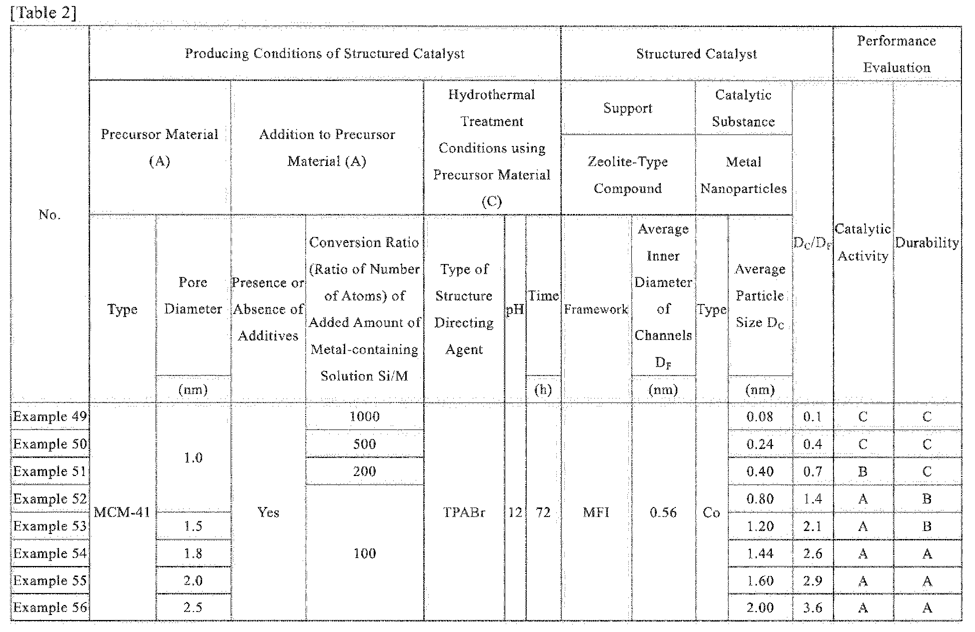

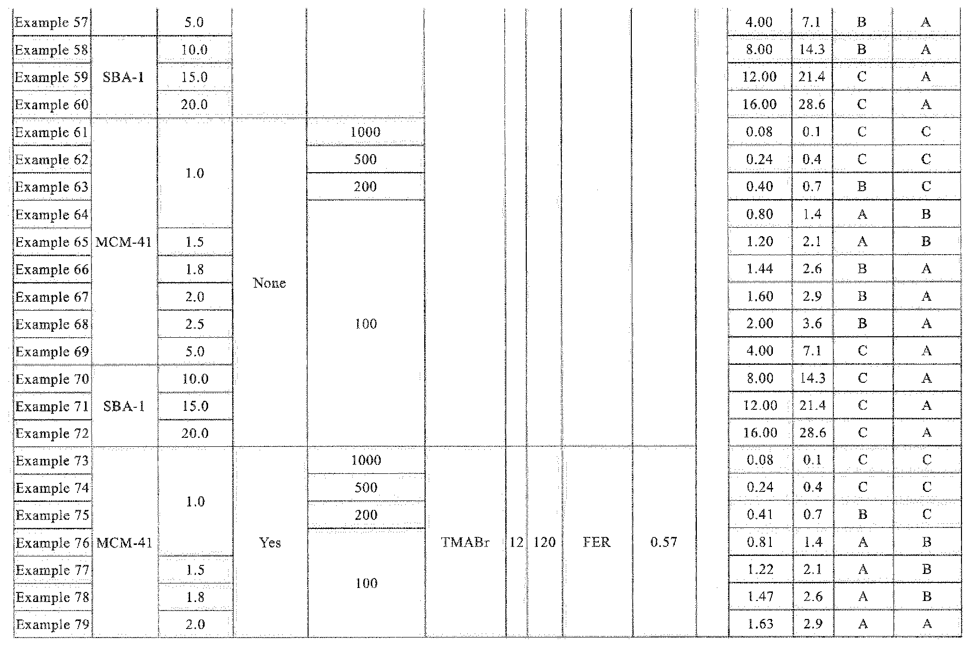

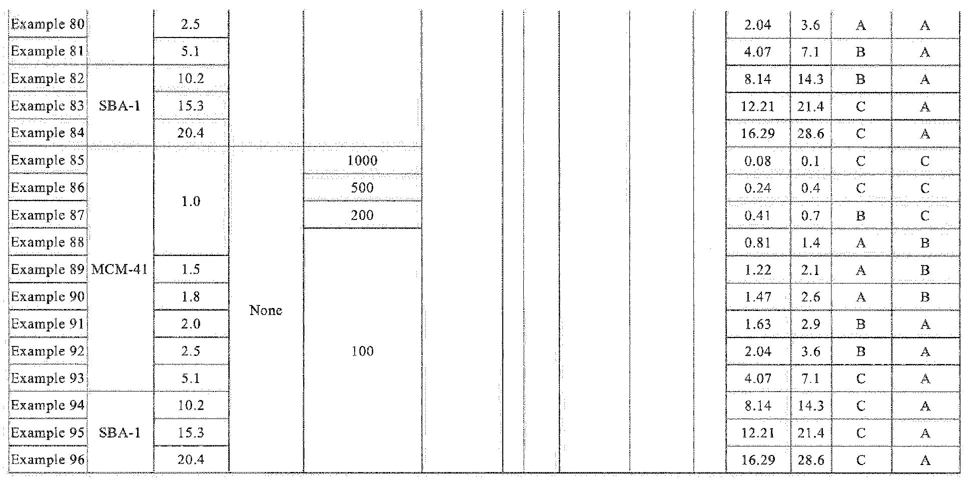

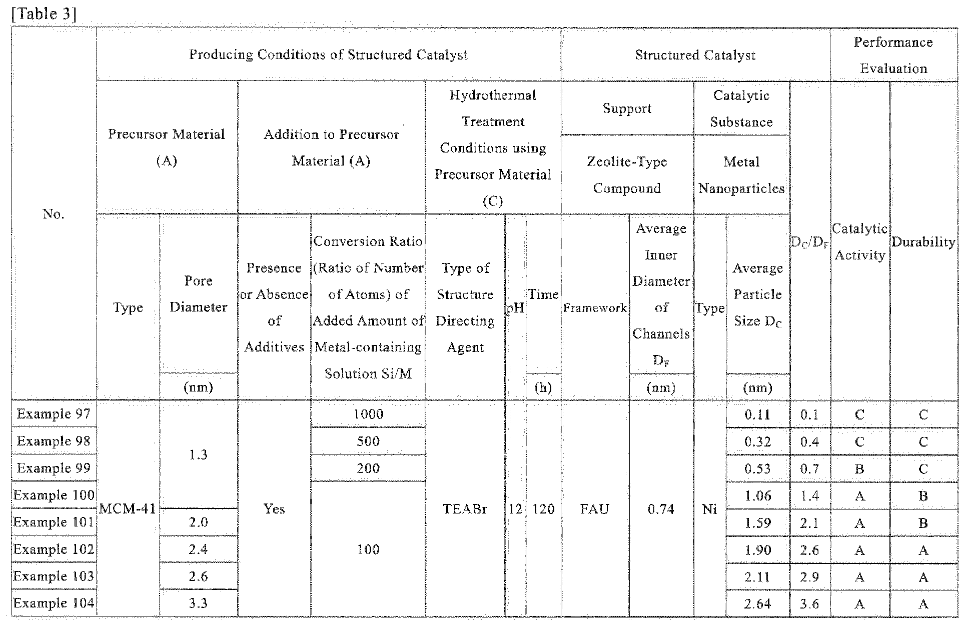

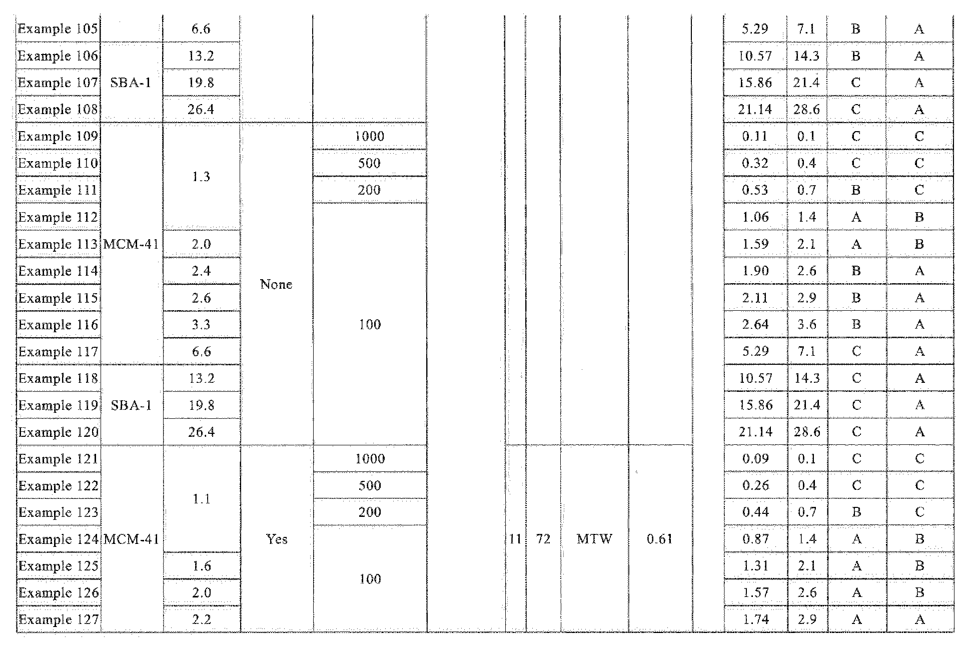

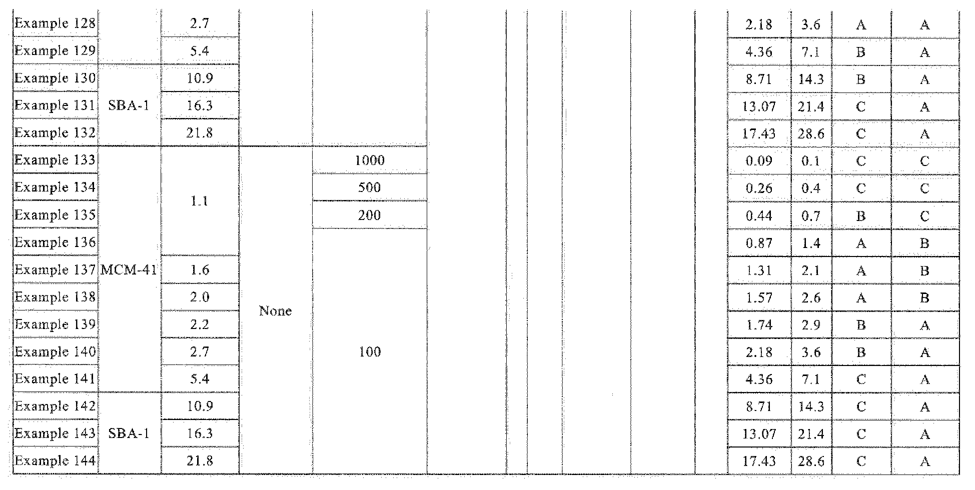

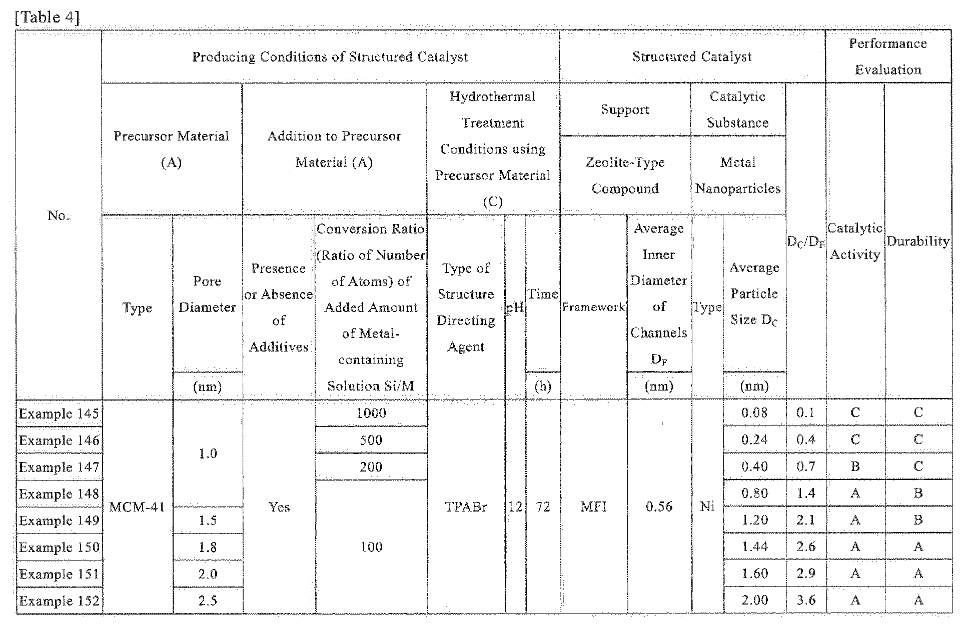

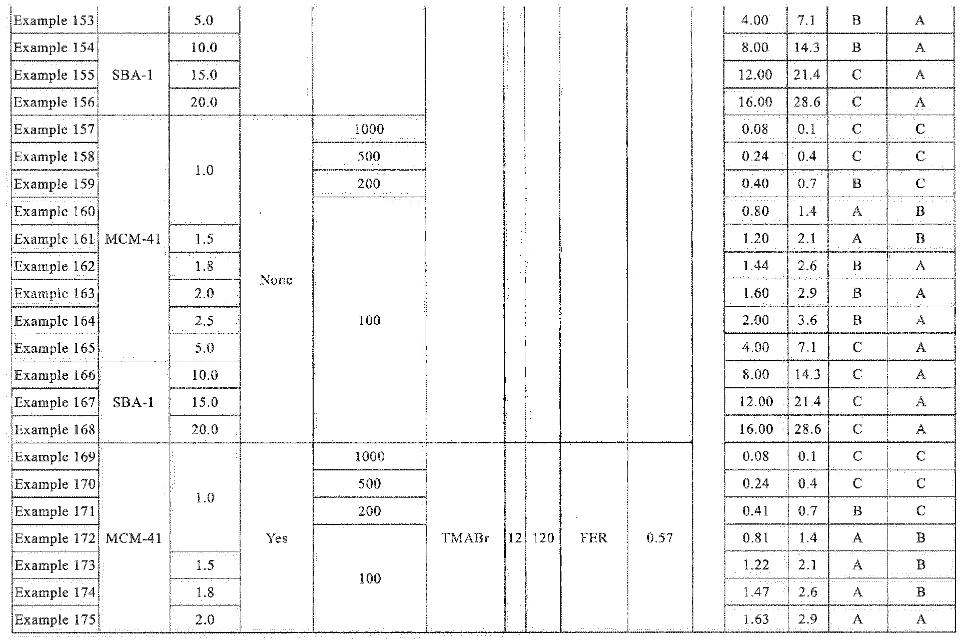

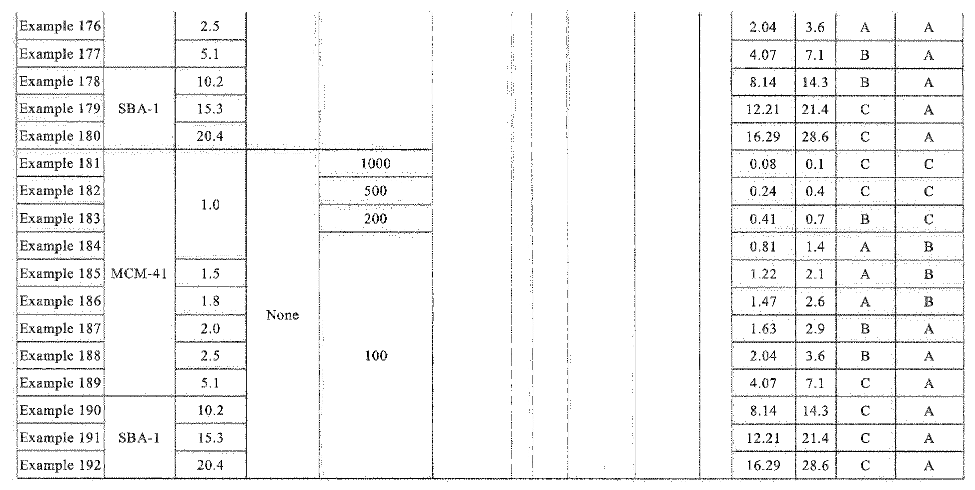

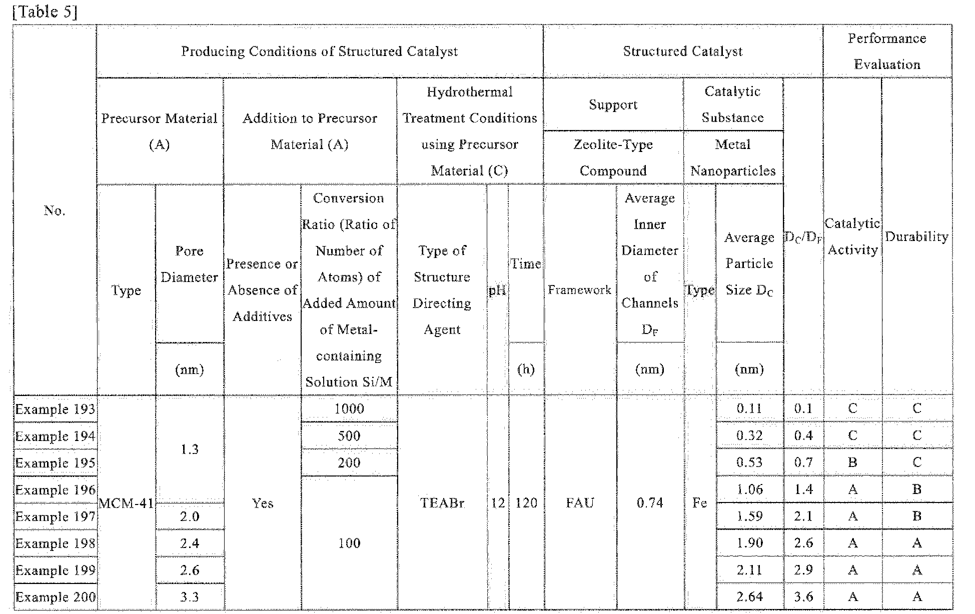

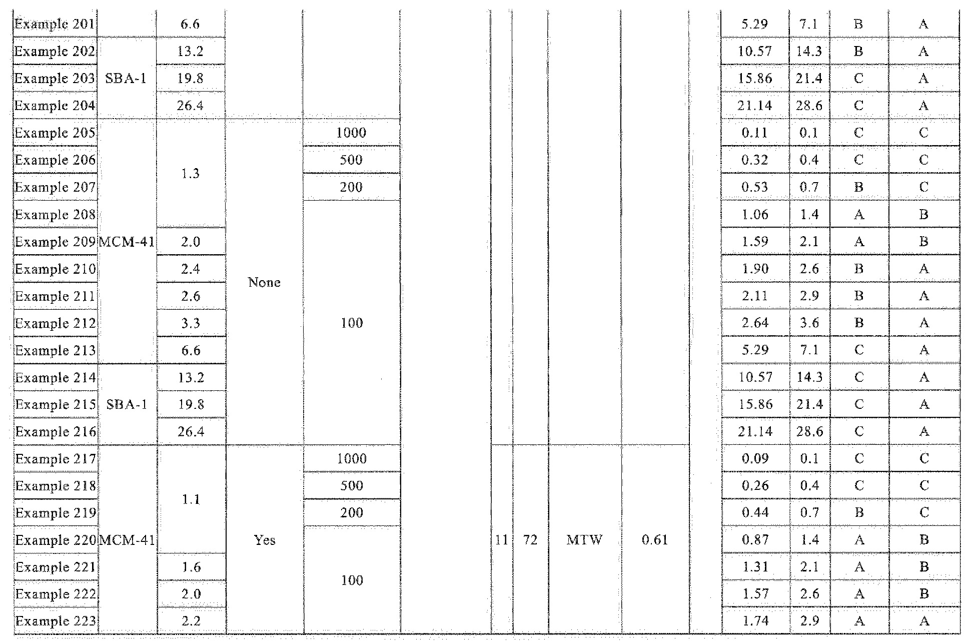

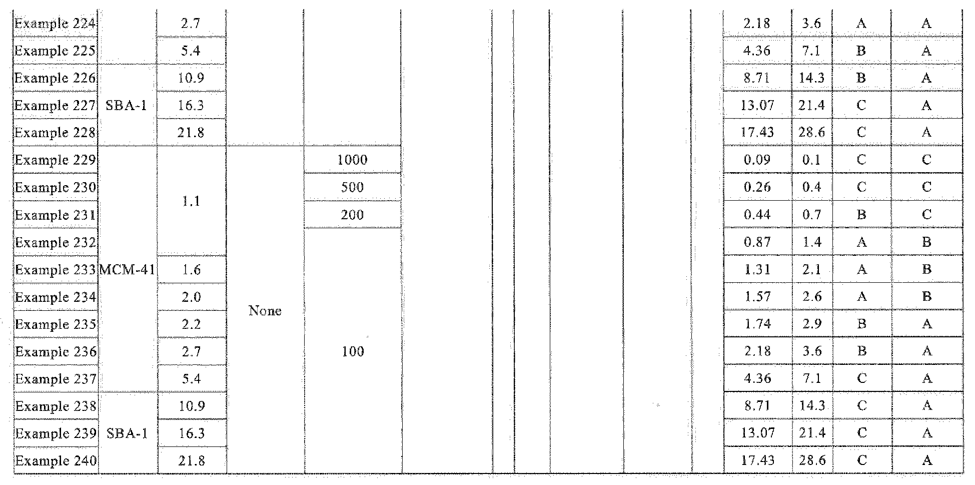

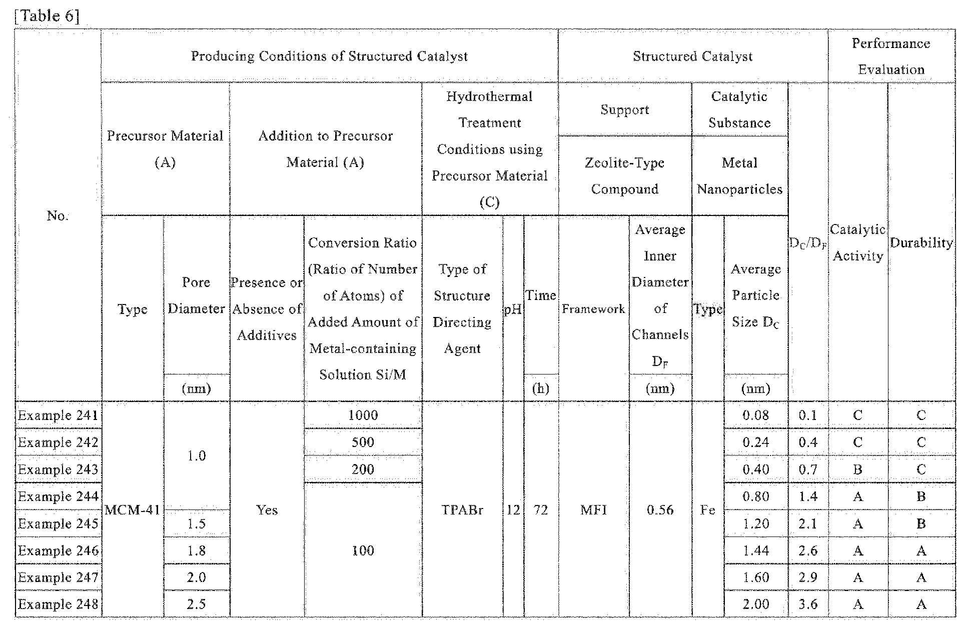

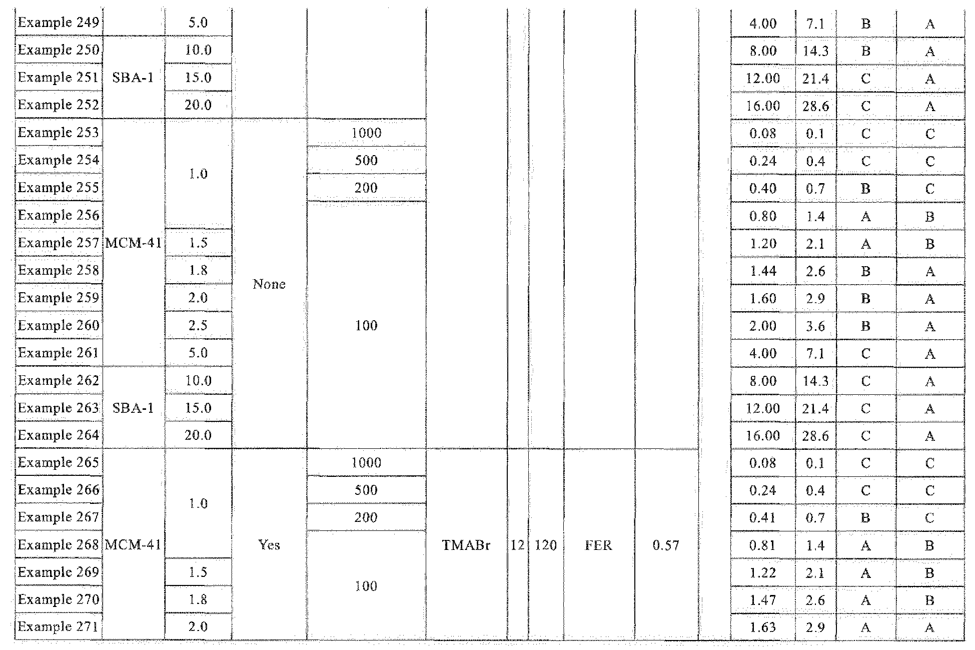

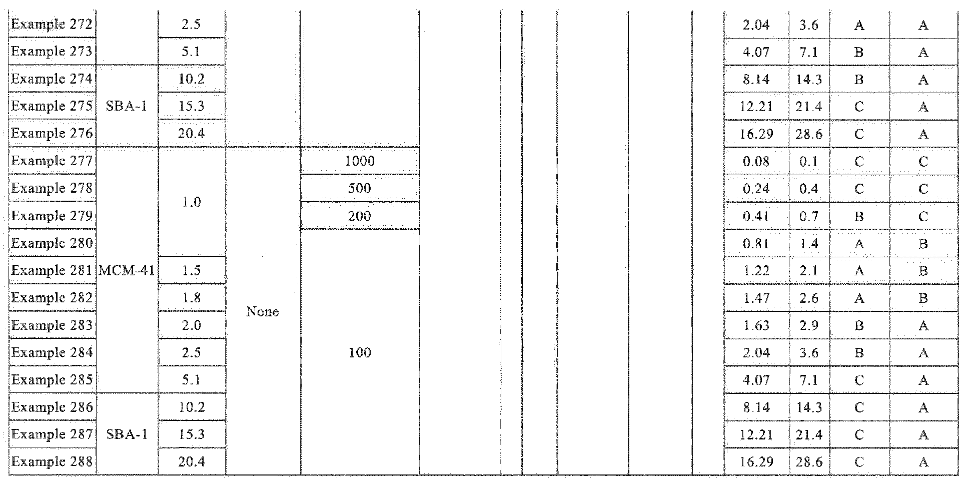

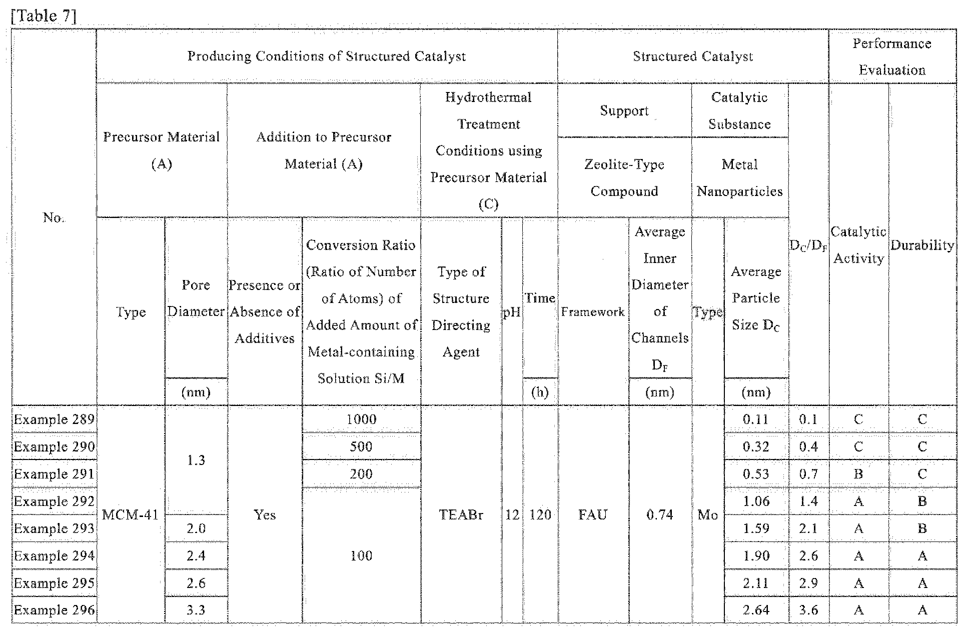

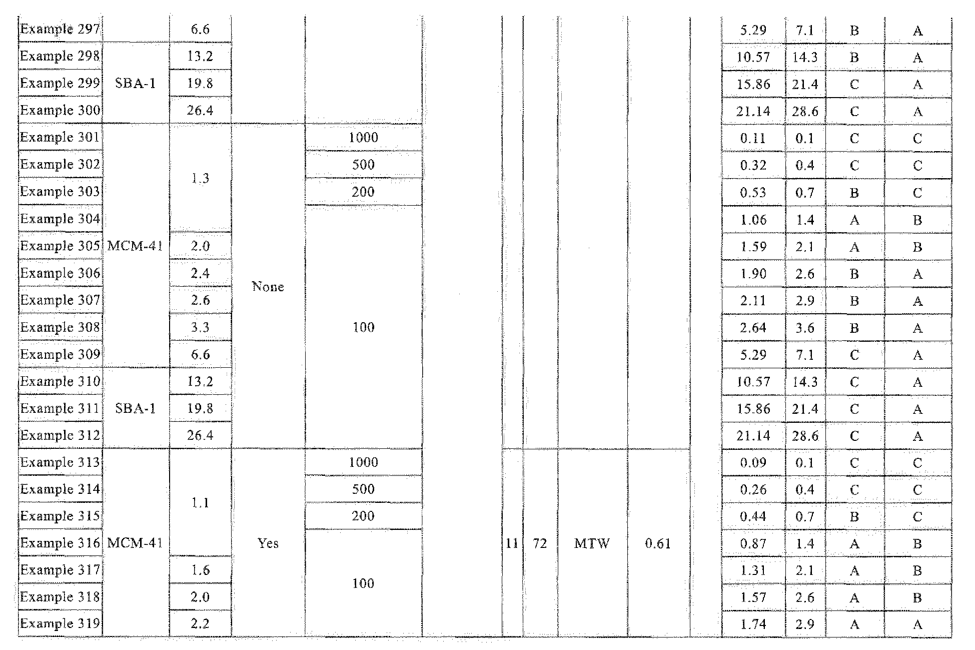

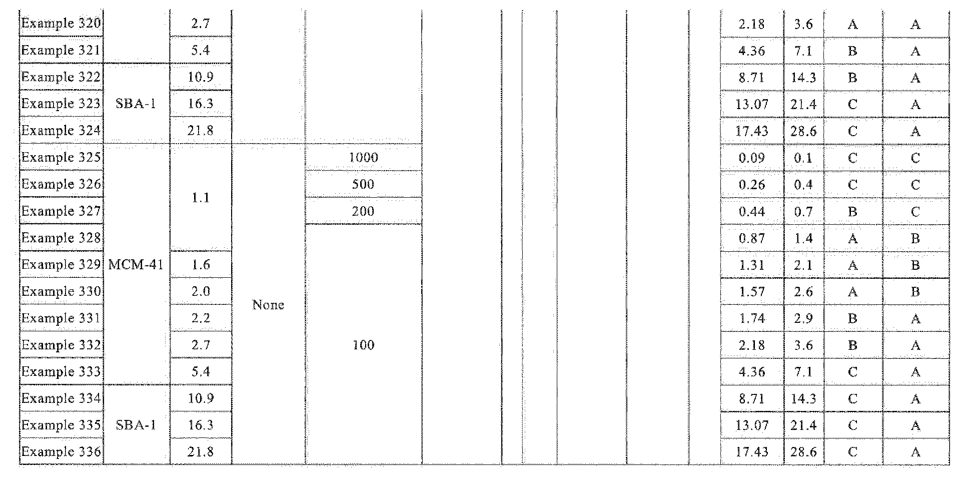

**[Table 8]**

| No. | Producing Conditions of Structured Catalyst | | | | | | | Structured Catalyst | | | | | Performance Evaluation | |
|---|---|---|---|---|---|---|---|---|---|---|---|---|---|---|
| | Precursor Material (A) | | Addition to Precursor Material (A) | | Hydrothermal Treatment Conditions using Precursor Material (C) | | | Support | | Catalytic Substance | | D_{C}/D_{F} | Catalytic Activity | Durability |
| | | | | | | | | Zeolite-Type Compound | | Metal Nanoparticles | | | | |
| | Type | Pore Diameter | Presence or Absence of Additives | Conversion Ratio (Ratio of Number of Atoms) of Added Amount of Metal-containing Solution Si/M | Type of Structure Directing Agent | pH | Time | Framework | Average Inner Diameter of Channels D_{F} | Type | Average Particle Size D_{C} | | | |
| | | (nm) | | | | | (h) | | (nm) | | (nm) | | | |
| Example 337 | MCM-41 | 1.0 | Yes | 1000 | TPABr | 12 | 72 | MFI | 0.56 | Mo | 0.08 | 0.1 | C | C |
| Example 338 | | | | 500 | | | | | | | 0.24 | 0.4 | C | C |
| Example 339 Example 340 | | | | 200 | | | | | | | 0.40 | 0.7 | B | C |
| | | | | 100 | | | | | | | 0.80 | 1.4 | A | B |
| Example 341 | | 1.5 | | | | | | | | | 1.20 | 2.1 | A | B |
| Example 342 | | 1.8 | | | | | | | | | 1.44 | 2.6 | A | A |
| Example 343 | | 2.0 | | | | | | | | | 1.60 | 2.9 | A | A |
| Example 344 | | 2.5 | | | | | | | | | 2.00 | 3.6 | A | A |
| Example 345 | | 5.0 | | | | | | | | | 4.00 | 7.1 | B | A |
| Example 346 | SBA-1 | 10.0 | | | | | | | | | 8.00 | 14.3 | B | A |
| Example 347 | | 15.0 | | | | | | | | | 12.00 | 21.4 | C | A |
| Example 348 | | 20.0 | | | | | | | | | 16.00 | 28.6 | C | A |
| Example 349 | MCM-41 | | None | 1000 | | | | | | | 0.08 | 0.1 | C | C |
| Example 350 | | 1.0 | | 500 | | | | | | | 0.24 | 0.4 | C | C |
| Example 351 | | | | 200 | | | | | | | 0.40 | 0.7 | B | C |
| Example 352 | | | | 100 | | | | | | | 0.80 | 1.4 | A | B |
| Example 353 | | 1.5 | | | | | | | | | 1.20 | 2.1 | A | B |
| Example 354 | | 1.8 | | | | | | | | | 1.44 | 2.6 | B | A |
| Example 355 | | 2.0 | | | | | | | | | 1.60 | 2.9 | B | A |
| Example 356 | | 2.5 | | | | | | | | | 2.00 | 3.6 | B | A |
| Example 357 | | 5.0 | | | | | | | | | 4.00 | 7.1 | C | A |
| Example 358 | SBA-1 | 10.0 | | | | | | | | | 8.00 | 14.3 | C | A |
| Example 359 | | 15.0 | | | | | | | | | 12.00 | 21.4 | C | A |
| Example 360 | | 20.0 | | | | | | | | | 16.00 | 28.6 | C | A |
| Example 361 | MCM-41 | | Yes | 1000 | TMABr | 12 | 120 | FER | 0.57 | | 0.08 | 0.1 | C | C |
| Example 362 | | 1.0 | | 500 | | | | | | | 0.24 | 0.4 | C | C |
| Example 363 | | | | 200 | | | | | | | 0.41 | 0.7 | B | C |
| Example 364 | | | | 100 | | | | | | | 0.81 | 1.4 | A | B |
| Example 365 | | 1.5 | | | | | | | | | 1.22 | 2.1 | A | B |
| Example 366 | | 1.8 | | | | | | | | | 1.47 | 2.6 | A | B |
| Example 367 | | 2.0 | | | | | | | | | 1.63 | 2.9 | A | A |
| Example 368 | | 2.5 | | | | | | | | | 2.04 | 3.6 | A | A |
| Example 369 | | 5.1 | | | | | | | | | 4.07 | 7.1 | B | A |
| Example 370 | SBA-1 | 10.2 | | | | | | | | | 8.14 | 14.3 | B | A |
| Example 371 | | 15.3 | | | | | | | | | 12.21 | 21.4 | C | A |
| Example 372 | | 20.4 | | | | | | | | | 16.29 | 28.6 | C | A |
| Example 373 | MCM-41 | | None | 1000 | | | | | | | 0.08 | 0.1 | C | C |
| Example 374 | | 1.0 | | 500 | | | | | | | 0.24 | 0.4 | C | C |
| Example 375 | | | | 200 | | | | | | | 0.41 | 0.7 | B | C |
| Example 376 | | | | 100 | | | | | | | 0.81 | 1.4 | A | B |
| Example 377 | | 1.5 | | | | | | | | | 1.22 | 2.1 | A | B |
| Example 378 | | 1.8 | | | | | | | | | 1.47 | 2.6 | A | B |
| Example 379 | | 2.0 | | | | | | | | | 1.63 | 2.9 | B | A |
| Example 380 | | 2.5 | | | | | | | | | 2.04 | 3.6 | B | A |
| Example 381 | | 5.1 | | | | | | | | | 4.07 | 7.1 | C | A |
| Example 382 | SBA-1 | 10.2 | | | | | | | | | 8.14 | 14.3 | C | A |
| Example 383 | | 15.3 | | | | | | | | | 12.21 | 21.4 | C | A |
| Example 384 | | 20.4 | | | | | | | | | 16.29 | 28.6 | C | A |
| Comparative Example 1 | - | | | | | | | MFI type silicalite | 0.56 | CoO x | ≤ 50 | ≤ 67.6 | C | D |
| Comparative Example 2 | - | | | | | | | MFI type silicalite | 0.56 | | | | D | D |

**[Table 9]**

| No. | Structured Catalyst | | | | | Performance Evaluation | | |
|---|---|---|---|---|---|---|---|---|
| | Support | | Catalytic Substance | | D_{C}/D_{F} | Catalytic Activity | Durability | Methane Dehydrogenaromatization Reaction |
| | Zeolite-Type Compound | | Metal Oxide Nanoparticles | | | | | |
| | Framework | Average Inner Diameter of Channels D_{F} | Type | Average Particle Size D_{C} | | | | |
| | | (nm) | | (nm) | | | | |
| Example 294 | FAU | 0.74 | Mo | 2.38 | 3.2 | A | A | B |
| Example 295 | | | | 2.64 | 3.6 | A | A | B |
| Example 296 | | | | 3.30 | 4.5 | A | A | B |
| Example 319 | MTW | 0.61 | | 2.18 | 3.6 | A | A | B |
| Example 320 | | | | 2.72 | 4.5 | A | A | B |
| Example 342 | MFI | 0.56 | | 1.80 | 3.2 | A | A | B |
| Example 343 | | 0.56 | | 2.00 | 3.6 | A | A | B |
| Example 344 | | 0.56 | | 2.50 | 4.5 | A | A | B |
| Example 367 | FER | 0.57 | | 2.04 | 3.6 | A | A | B |
| Example 368 | | 0.57 | | 2.54 | 4.5 | A | A | B |

As can be seen from Tables 1 to 8, the catalytic structural bodies (Examples 1 to 384), which were confirmed by cross-sectional observation to hold the catalytic substance in the support were found to exhibit excellent catalytic activity in the decomposition reaction of butylbenzene and excellent durability as a catalyst compared to the structured catalyst in which the catalytic substance was simply adhered to the outer surface of the support (Comparative Example 1) or the skeletal body itself having no functional substance (Comparative Example 2).

In addition, the relationship between the amount of metal (mass%) embedded in the support of the structured catalyst measured in the evaluation [C]; and the yield (mol%) of a compound having a molecular weight smaller than that of butylbenzene contained in the produced liquid was evaluated. The evaluation method was the same as the evaluation method performed in "(1) catalytic activity" in the [D] "performance evaluation" described above.

As a result, in each example, when the added amount of the metal-containing solution added to the precursor material (A), converted into the ratio of number of atoms Si/M (M = Fe), was from 50 to 200 (the content of the metal oxide nanoparticles relative to the structured catalyst being from 0.5 to 2.5 mass%), the yield of the compound having a molecular weight lower than that of butylbenzene contained in the product liquid was 32 mol% or greater, and the catalytic activity in the decomposition reaction of butylbenzene was found to be greater than or equal to the pass level.

On the other hand, in the silicalite of Comparative Example 1 in which the catalytic substance was attached only to the outer surface of the support, the catalytic activity in the decomposition reaction of butylbenzene was improved compared to the support of Comparative Example 2, which had no catalytic substance, but inferior durability as a catalyst was exhibited compared to the structured catalyst of Examples 1 to 384.

In addition, the support of Comparative Example 2, which had no catalytic substance, exhibited little catalytic activity in the decomposition reaction of butylbenzene, and both the catalytic activity and the durability were inferior compared to the structured catalyst of Examples 1 to 384.

Furthermore, Table 9 indicates that the structured catalyst containing molybdenum not only exhibited excellent catalytic activity and durability described above but also achieved a good result in the methane dehydroaromatization reaction. In this way, the structured catalyst according to the present example, in a dehydrocyclization reaction of a light hydrocarbon, can produce an aromatic hydrocarbon. Moreover, it was confirmed that excellent catalytic activity and durability as described above were also achieved when the structured catalyst was used to produce an aromatic hydrocarbon.

A method for producing an aromatic hydrocarbon from a methane-containing gas using a catalyst, the catalyst including a support of a porous framework composed of a zeolite-type compound and at least one metal nanoparticle present in the support, the support having channels communicating with each other, the metal nanoparticles including a structured catalyst held in at least an enlarged pore portion of the channels of the support.

### Reference Signs List

1 Structured catalyst
10 Support
10a Outer surface
11 Channel
11a Pore
12 Enlarged pore portion
20 Catalytic substance
30 Catalytic substance

## Claims

1. A structured catalyst for aromatic hydrocarbon production, comprising:
a support of a porous framework composed of a zeolite-type compound; and
at least one catalytic substance present in the support,
the support comprising channels communicating with each other, and
the catalytic substance being made of metal nanoparticles and being present at least in the channels of the support.

2. The structured catalyst for aromatic hydrocarbon production according to claim 1, wherein the metal nanoparticles are nanoparticles composed of at least one metal selected from the group consisting of cobalt (Co), nickel (Ni), iron (Fe), copper (Cu), gold (Au), silver (Ag), platinum (Pt), palladium (Pd), rhodium (Rd), iridium (Ir), ruthenium (Ru), osmium (Os), and molybdenum (Mo).

3. The structured catalyst for aromatic hydrocarbon production according to claim 1 or 2, wherein the channels each comprise an enlarged pore portion, and the catalytic substance is at least embedded in the enlarged pore portion.

4. The structured catalyst for aromatic hydrocarbon production according to claim 2, wherein the enlarged pore portion communicates with a plurality of pores constituting any one of a one-dimensional pore, a two-dimensional pore, and a three-dimensional pore.

5. The structured catalyst for aromatic hydrocarbon production according to claim 3 or 4, wherein the average particle size of the metal nanoparticles is greater than an average inner diameter of the channels and is less than or equal to an inner diameter of the enlarged pore portion.

6. The structured catalyst for aromatic hydrocarbon production according to any one of claims 1 to 5, wherein a metal element (M) of the metal nanoparticles is contained in an amount of from 0.5 to 2.5 mass% relative to the structured catalyst for aromatic hydrocarbon production.

7. The structured catalyst for aromatic hydrocarbon production according to any one of claims 1 to 6, wherein the average particle size of the metal nanoparticles is from 0.08 nm to 30 nm.

8. The structured catalyst for aromatic hydrocarbon production according to claim 7, wherein the average particle size of the metal nanoparticles is from 0.35 nm to 11.0 nm.

9. The structured catalyst for aromatic hydrocarbon production according to any one of claims 1 to 8, wherein the ratio of the average particle size of the metal nanoparticles to the average inner diameter of the channels is from 0.05 to 300.

10. The structured catalyst for aromatic hydrocarbon production according to claim 9, wherein the ratio of the average particle size of the metal nanoparticles to the average inner diameter of the channels is from 0.1 to 30.

11. The structured catalyst for aromatic hydrocarbon production according to claim 9 or 10, wherein the ratio of the average particle size of the metal nanoparticles to the average inner diameter of the channels is from 1.4 to 3.6.

12. The structured catalyst for aromatic hydrocarbon production according to any one of claims 1 to 11, wherein
the channels comprise any one of the one-dimensional pore, the two-dimensional pore, and the three-dimensional pore defined by a framework of the zeolite-type compound and the enlarged pore portion which is different from any one of the one-dimensional pore, the two-dimensional pore, and the three-dimensional pore,
the average inner diameter of the channels is from 0.1 nm to 1.5 nm, and
the inner diameter of the enlarged pore portion is from 0.5 nm to 50 nm.

13. The structured catalyst for aromatic hydrocarbon production according to any one of claims 1 to 12, further comprising at least one other catalytic substance held on an outer surface of the support.

14. The structured catalyst for aromatic hydrocarbon production according to claim 13, wherein the content of the at least one catalytic substance present in the support is greater than that of the at least one other catalytic substance held on the outer surface of the support.

15. The structured catalyst for aromatic hydrocarbon production according to any one of claims 1 to 14, wherein the zeolite-type compound is a silicate compound.

16. An aromatic hydrocarbon producing device comprising a structured catalyst for aromatic hydrocarbon production described in any one of claims 1 to 15.

17. A method for producing a structured catalyst for aromatic hydrocarbon production, comprising:
a step of calcination of calcinating a precursor material (B) obtained by impregnating a precursor material (A), for obtaining a support of a porous framework composed of zeolite-type compound, with a metal-containing solution;
a step of hydrothermal treatment of hydrothermal-treating a precursor material (C) obtained by calcinating the precursor material (B); and
a step of reduction treatment of the precursor material (C) that is hydrothermal-treated.

18. The method for producing a structured catalyst for aromatic hydrocarbon production according to claim 17, wherein from 50 to 500 mass% of a nonionic surfactant is added to the precursor material (A) before the step of calcination.

19. The method for producing a structured catalyst for aromatic hydrocarbon production according to claim 17 or 18, wherein the precursor material (A) is impregnated with the metal-containing solution by adding the metal-containing solution to the precursor material (A) in multiple portions before the step of calcination.

20. The method for producing a structured catalyst for aromatic hydrocarbon production according to any one of claims 17 to 19, wherein in impregnating the precursor material (A) with the metal-containing solution before the step of calcination, an added amount of the metal-containing solution added to the precursor material (A), converted into a ratio of silicon (Si) constituting the precursor material (A) to a metal element (M) included in the metal-containing solution added to the precursor material (A), a ratio of number of atoms Si/M, is adjusted to from 10 to 1000.

21. The method for producing a structured catalyst for aromatic hydrocarbon production according to claim 17, wherein in the step of hydrothermal treatment, the precursor material (C) and a structure directing agent are mixed.

22. The method for producing a structured catalyst for aromatic hydrocarbon production according to claim 17, wherein the step of hydrothermal treatment is performed in basic condition.

23. The method of producing a structured catalyst for aromatic hydrocarbon production according to claim 17, further comprising a step of carbonizing the structured catalyst for aromatic hydrocarbon production.

24. A method for producing an aromatic hydrocarbon, comprising adding a methane-containing gas to a structured catalyst for aromatic hydrocarbon production according to any one of claims 1 to 12.

25. The method for producing an aromatic hydrocarbon according to claim 24, comprising
the step of carbonizing the metal nanoparticles; and
a step of adding a methane-containing gas to the structured catalyst.

26. A method for producing an aromatic hydrocarbon, comprising treating a methane-containing gas with the aromatic hydrocarbon producing device described in claim 16.
